# EUROPEAN PATENT SPECIFICATION

(11) **EP 4 221 626 B1**
(45) Date of publication and mention of the grant of the patent: **29.07.2026**
(21) Application number: 21874683.2
(22) Date of filing: 27.09.2021
(51) Int. Cl.: A61B 34/30, A61B 17/34, A61B 50/20, A61B 90/50, A61B 50/33, A61B 90/57

(54) **ROBOTIC INSTRUMENT ALIGNMENT**
AUSRICHTUNG VON ROBOTERINSTRUMENTEN
ALIGNEMENT D'INSTRUMENT ROBOTIQUE

(30) Priority: 30.09.2020 US 202063085602 P
(43) Date of publication of application: 09.08.2023
(73) Proprietor: Auris Health, Inc., Santa Clara, CA 95054 (US)
(72) Inventor: FAJARDO VARGAS, Javier O., Redwood City, California 94065 (US); MA, Polly Charlene, Redwood City, California 94065 (US); TANG, Jordan J., Redwood City, California 94065 (US)
(74) Representative: Carpmaels & Ransford LLP
(86) International application number: PCT/IB2021/058786
(87) International publication number: WO 2022/070016

(56) References cited:
- EP-A1- 3 666 215
- EP-B1- 3 232 957
- EP-B1- 3 307 369
- US-A1- 2011 071 543
- US-A1- 2015 223 832
- US-A1- 2017 303 940
- US-A1- 2020 000 537
- US-A1- 2020 000 537
- US-A1- 2020 121 405
- US-A1- 2020 206 472
- US-B2- 10 470 827

## Description

### RELATED APPLICATION(S)

This application claims priority to U.S. Provisional Application No. 63/085,602, filed September 30, 2020, and entitled ROBOTIC INSTRUMENT ALIGNMENT.

### BACKGROUND

### Field

The present disclosure relates to the field of medical procedures and devices.

### Description of Related Art

Various medical procedures involve the use of one or more medical devices for accessing a target anatomical site in a patient. In some instances, the improper alignment of certain devices when accessing the site in connection with a procedure can adversely affect the health of the patient, the integrity of the medical device(s), and/or the efficacy of the procedure.

US 2020/121405 A1 discusses a robotic surgical system including a surgical robot and a guide tube. The surgical robot includes a robot arm and a controller. The controller is configured to establish a software-based remote center of motion of a surgical instrument attached to the robot arm based on a location of a surgical portal in a patient through which the surgical instrument is inserted. The guide tube having a trailing end supported by the robot arm of the surgical robot, a leading end inserted in the surgical portal and maintaining alignment between the robotic arm and the surgical portal during a surgical instrument exchange, and an elongated tubular body through which an elongated shaft of the surgical instrument is inserted or removed during the surgical instrument exchange.

EP 3 666 215 A1 discusses a cannula for a surgical system which may include a magnet located in a position to be sensed by the surgical system in a mounted position of the cannula to the surgical system. At least one of a presence of the magnet and a polarity of the magnet is sensed in the mounted position of the cannula to provide identification information relating to the cannula. Exemplary embodiments further encompass a patient side cart for a teleoperated surgical system, the patient side cart including a base, a main column, and an arm connected to the main column. The arm may include a mount to receive a cannula and a reader to sense a magnet of an identification device in the cannula so as to receive identification information relating to a mounted cannula..

### SUMMARY

The invention is defined by claims 1 and 14. Further embodiments of the invention are defined by the dependent claims. Described herein are systems, devices, and methods to facilitate alignment of medical devices/instruments in connection with certain medical procedures. In particular, systems, devices, and methods in accordance with one or more aspects of the present disclosure can facilitate alignment of aspiration catheter devices and/or other types of medical/surgical instruments and/or systems with percutaneous-access devices/sheaths, which may advantageously reduce the risk of damage to patient anatomy and/or medical equipment.

In some implementations, the present disclosure relates to an alignment device comprising an elongate rod, a sealing hub coupled to a proximal end of the elongate rod and configured to form a fluid-tight seal within a portion of a percutaneous-access device, and an alignment structure coupled to the sealing hub and having a form configured to engage with a component of an instrument manipulator assembly of a robotic system.

The elongate rod has may have a blunt distal end. In some embodiments, a distal end of the elongate rod includes a flat axial face portion and a rounded circumferential edge portion. The alignment structure can have a concave proximal surface conforming to an outer surface of the instrument manipulator assembly. For example, the alignment structure can have a vertical-alignment lip that is inwardly-projecting and circumferential with respect to a curvature of the concave proximal surface. The vertical-alignment lip may be configured to be placed against at least one of an upward-facing surface or a downward-facing surface of the component of the instrument manipulator assembly when the concave proximal surface is placed against the outer surface of the instrument manipulator assembly.

In some embodiments, the alignment structure includes one or more alignment markings indicating an alignment position of the alignment structure relative to at least one component of the instrument manipulator assembly. The sealing hub may have external threading configured to engage with internal threading of the percutaneous-access device.

The alignment structure may be configured to be attached to the sealing hub from a state separate from the sealing hub. For example, the alignment structure comprises a distally-projecting male connector configured to be coupled to the sealing hub. The alignment structure can be axially rotatable relative to the sealing hub.

In some implementations, the present disclosure relates to an alignment device comprising an atraumatic contact means configured to project from a distal portion of a sheath device, a sealing means configured to be coupled to a proximal portion of the sheath device and form a fluid-tight seal between the sheath device and the sealing means, and an alignment means coupled to the sealing means and configured to facilitate alignment of one or more components of the alignment device with one or more components associated with a robot arm of a robotic system.

The atraumatic contact means can comprise an elongate, solid rod having a blunt distal end. In some embodiments, the sealing means comprises a hub structure having a tapered surface configured to be placed against a corresponding interior surface of the sheath device to form the fluid-tight seal. The alignment means can comprise a concave dish form.

In some implementations, the present disclosure relates to a robotic system comprising a robotic arm, an instrument manipulator assembly associated with a distal portion of the robotic arm, a percutaneous-access device comprising an elongate shaft forming an access lumen, and an alignment device including an elongate rod configured to be disposed at least partially within the access lumen and a proximal alignment member having a form configured to engage with an outer surface of the instrument manipulator assembly.

In some embodiments, the instrument manipulator assembly includes an adapter component, the outer surface being associated with a rotating plate of the adapter component. For example, the adapter component can be coupled to a drape that is configured to provide a sterile barrier between the robotic arm and an instrument of the instrument manipulator assembly that is coupled to the adapter component. The robotic system can further comprise a catheter assembly including a catheter coupled to a catheter handle configured to be attached to the adapter component.

The alignment device may include a hub member secured to a proximal end of the elongate rod. For example, the alignment member can be secured to the alignment device on a proximal side of the hub member. In some embodiments, a top surface of the alignment member has one or more alignment markers thereon. The robotic arm may be configured to move along an alignment path that is based on kinematic data relating to a position of the robotic arm when the instrument manipulator assembly is engaged with the alignment member of the alignment device.

In some implementations, the present disclosure relates to a method of aligning a robotic arm. The method comprises manipulating a robotic arm of a robotic system to an alignment position in which a portion of an instrument manipulator assembly associated with a distal portion of the robotic arm is in engagement with an alignment structure of an alignment device having an elongate rod disposed at least in part within a lumen of an access device, withdrawing the elongate rod from the lumen, and advancing the instrument manipulator assembly along an alignment path based on the alignment position to advance an instrument coupled to the instrument manipulator assembly through the lumen of the access device.

The method can further comprise determining an alignment axis of the lumen of the access device based on the alignment position. The method can further comprise determining the alignment path based on kinematic robotic arm data associated with the alignment position. Advancing the instrument manipulator assembly along the alignment path can involve constraining movement of the robotic arm to the alignment path.

In some embodiments, the method further comprises axially rotating a plate component of the instrument manipulator assembly to bring one or more markings on the plate component into alignment with one or more corresponding markings associated with the alignment structure of the alignment device. The method can further comprise, after said withdrawing the elongate rod from the lumen, inserting an inner sheath component at least partially into the lumen of the access device, the inner sheath component forming a proximal seal with the access device.

The method may further comprise engaging a hub component of the alignment device with a proximal portion of the access device to form a fluid seal. For example, the alignment structure may be coupled to the hub component. The method may further comprise aspirating fluid from an anatomical site through the instrument, the instrument being an aspiration catheter.

**In** some implementations, the present disclosure relates to a percutaneous-access kit comprising a catheter coupled to a catheter handle, a percutaneous-access sheath having an access lumen, and a catheter-alignment device including an elongate rod and a proximal alignment structure.

In some embodiments, the percutaneous-access kit further comprises a tray structure including one or more wells configured to have the catheter, the catheter handle, the percutaneous-access sheath, and the catheter-alignment device disposed at least partially therein. The percutaneous-access kit can further comprise an inner sheath configured to be disposed at least partially within the access lumen of the percutaneous-access sheath and form a proximal fluid seal between a hub of the inner sheath and a hub of the percutaneous-access sheath. The percutaneous-access kit can further comprise an elongate dilator form.

The catheter-alignment device can further include a proximal hub configured to form a proximal fluid seal between the proximal hub and a hub of the percutaneous-access sheath. In some embodiments, the elongate rod and the proximal hub are assembled together in the percutaneous-access kit. In some embodiments, the alignment structure is separate from the elongate rod and the proximal hub in the percutaneous-access kit.

For purposes of summarizing the disclosure, certain aspects, advantages and novel features have been described. It is to be understood that not necessarily all such advantages may be achieved in accordance with any particular embodiment. Thus, the disclosed embodiments may be carried out in a manner that achieves or optimizes one advantage or group of advantages as taught herein without necessarily achieving other advantages as may be taught or suggested herein.

### BRIEF DESCRIPTION OF THE DRAWINGS

Various embodiments are depicted in the accompanying drawings for illustrative purposes and should in no way be interpreted as limiting the scope of the inventions. In addition, various features of different disclosed embodiments can be combined to form additional embodiments, which are part of this disclosure. Throughout the drawings, reference numbers may be reused to indicate correspondence between reference elements.
Figure 1 illustrates an embodiment of a robotic medical system including one or more percutaneous-access devices in accordance with one or more embodiments.
Figure 2 illustrates a robotic system arranged for diagnostic and/or therapeutic bronchoscopy in accordance with one or more embodiments.
Figure 3 illustrates a table-based robotic system in accordance with one or more embodiments.
Figure 4 illustrates medical system components that may be implemented in any of the medical systems of Figures 1-3 in accordance with one or more embodiments.
Figures 5A and 5B show side and cross-sectional views, respectively, of a patient in a prone position having a percutaneous-access device or system disposed at least partially in his/her renal anatomy in accordance with one or more embodiments.
Figures 6A and 6B show side and cross-sectional views, respectively, of a patient in a modified supine position having a percutaneous-access device or system disposed at least partially in his/her renal anatomy in accordance with one or more embodiments.
Figures 7A and 7B show isomeric and side views, respectively, of inner and outer sheath components of a percutaneous-access device in accordance with one or more embodiments.
Figures 8A and 8B show perspective and side cut-away close-up views, respectively of a percutaneous-access system disposed at least partially in portions of the renal anatomy of a patient in accordance with one or more embodiments.
Figure 9 shows an exploded view of an instrument device manipulator assembly associated with a robotic arm in accordance with one or more embodiments.
Figure 10 shows a distal and side isometric view of an alignment device in accordance with one or more embodiments.
Figure 11 shows a proximal and side isometric view of a proximal portion of the alignment device of Figure 10 in accordance with one or more embodiments.
Figure 12 shows a side view of an alignment device disposed in a percutaneous-access device in accordance with one or more embodiments.
Figure 13 shows a cut-away view of a proximal portion of the alignment device of Figure 12 in accordance with one or more embodiments.
Figure 14 shows an alignment device having an alignment structure engaged with one or more surfaces and/or features of an instrument device manipulator assembly in accordance with one or more embodiments.
Figure 15 shows a kit for a percutaneous-access system in accordance with one or more embodiments.
Figures 16-1, 16-2, 16-3, 16-4, and 16-5 show a flow diagram illustrating a process for aligning a medical instrument in accordance with one or more embodiments.
Figures 17-1, 17-2, 17-3, 17-4, and 17-5 show certain images corresponding to various blocks, states, and/or operations associated with the process of Figures 16-1, 16-2, 16-3, 16-4, and 16-5, respectively, in accordance with one or more embodiments.

### DETAILED DESCRIPTION

The headings provided herein are for convenience only and do not necessarily affect the scope or meaning of the claimed invention. Although certain preferred embodiments and examples are disclosed below, inventive subject matter extends beyond the specifically disclosed embodiments to other alternative embodiments and/or uses and to modifications and equivalents thereof. Thus, the scope of the claims that may arise herefrom is not limited by any of the particular embodiments described below. For example, in any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and/or devices described herein may be embodied as integrated components or as separate components. For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

Although certain spatially relative terms, such as "outer," "inner," "upper," "lower," "below," "above," "vertical," "horizontal," "top," "bottom," and similar terms, are used herein to describe a spatial relationship of one device/element or anatomical structure to another device/element or anatomical structure, it is understood that these terms are used herein for ease of description to describe the positional relationship between element(s)/structures(s), as illustrated in the drawings. It should be understood that spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa. It should be understood that spatially relative terms, including those listed above, may be understood relative to a respective illustrated orientation of a referenced figure.

Certain reference numbers are re-used across different figures of the figure set of the present disclosure as a matter of convenience for devices, components, systems, features, and/or modules having features that are similar in one or more respects. However, with respect to any of the embodiments disclosed herein, re-use of common reference numbers in the drawings does not necessarily indicate that such features, devices, components, or modules are identical or similar. Rather, one having ordinary skill in the art may be informed by context with respect to the degree to which usage of common reference numbers can imply similarity between referenced subject matter. Use of a particular reference number in the context of the description of a particular figure can be understood to relate to the identified device, component, aspect, feature, module, or system in that particular figure, and not necessarily to any devices, components, aspects, features, modules, or systems identified by the same reference number in another figure. Furthermore, aspects of separate figures identified with common reference numbers can be interpreted to share characteristics or to be entirely independent of one another.

The present disclosure relates to systems, devices, and methods for aligning devices/instruments of a percutaneous-access system, such as catheters or other instruments, with certain percutaneous-access devices. With respect to percutaneous-access devices and other medical devices relevant to the present disclosure, the term "device" is used according to its broad and ordinary meaning and may refer to any type of tool, instrument, assembly, system, apparatus, component, or the like. In some contexts herein, the term "instrument" may be used substantially interchangeably with the term "device."

Although certain aspects of the present disclosure are described in detail herein in the context of renal, urological, and/or nephrological procedures, such as kidney stone removal/treatment procedures, it should be understood that such context is provided for convenience and clarity, and percutaneous-access, device-alignment, and fluid-management concepts disclosed herein are applicable to any suitable medical procedures, such as robotic bronchoscopy. However, as mentioned, description of the renal/urinary anatomy and associated medical issues and procedures is presented below to aid in the description of the inventive concepts disclosed herein.

Kidney stone disease, also known as urolithiasis, is a medical condition that involves the formation in the urinary tract of a solid piece of material, referred to as "kidney stones," "urinary stones," "renal calculi," "renal lithiasis," or "nephrolithiasis." Urinary stones may be formed and/or found in the kidneys, the ureters, and the bladder (referred to as "bladder stones"). Such urinary stones can form as a result of mineral concentration in urinary fluid and can cause significant abdominal pain once such stones reach a size sufficient to impede urine flow through the ureter or urethra. Urinary stones may be formed from calcium, magnesium, ammonia, ur acid, cysteine, and/or other compounds or combinations thereof.

Generally, there are several methods for treating patients with kidney stones, including observation, medical treatments (such as expulsion therapy), non-invasive treatments (such as extracorporeal shock wave lithotripsy (ESWL), and surgical treatments (such as ureteroscopy and percutaneous nephrolithotomy ("PCNL"); see description below for details). In surgical approaches (e.g., ureteroscopy and PCNL), the physician gains access to the pathology (i.e., the object to be removed; e.g., the stone), the stone is broken into smaller pieces or fragments, and the relatively small stone fragments/particulates are mechanically extracted from the kidney.

In some procedures, surgeons may insert a ureteroscope into the urinary tract through the urethra to remove urinary stones from the bladder and ureter. Typically, a ureteroscope includes an endoscope at its distal end configured to enable visualization of the urinary tract. The ureteroscope can also include a lithotripsy device to capture or break apart urinary stones. During a ureteroscopy procedure, one physician/technician may control the position of the ureteroscope, while another other physician/technician may control the lithotripsy device(s).

In some procedures, such as procedures for removing relatively large stones, physicians may use a percutaneous nephrolithotomy ("PCNL") technique that involves inserting a nephroscope through the skin (i.e., percutaneously) and intervening tissue to provide access to the treatment site for breaking-up and/or removing the stone(s). A percutaneous-access device (e.g., nephroscope, sheath, sheath assembly, and/or catheter) used to provide an access channel to the target anatomical site (and/or a direct-entry endoscope) may include one or more fluid channels for providing irrigation fluid flow to the target site and/or aspirating fluid from the target site (e.g., through passive outflow and/or active suction). Generally, the efficacy of percutaneous access to a target calyx and/or direct access to the target calyx through the urinary system can depend at least in part on the fluid conditions (e.g., fluid volume and/or pressure levels) within the kidney. Such conditions can depend at least in part on the fluid management provided using the fluid channel(s), wherein such fluid management is directed at least in part by certain control circuitry associated with one or more devices/systems implemented in a medical procedure. During PCNL, fluidics can be applied to clear stone dust, small fragments, and thrombus from the treatment site as well as the visual field.

Robotic-assisted percutaneous procedures can be implemented in connection with various medical procedures, such as kidney stone removal procedures, wherein robotic tools can enable a physician/urologist to perform endoscopic (e.g., ureteroscopy) target access as well as percutaneous access/treatment. Advantageously, aspects of the present disclosure relate to systems, devices, and methods for aligning robotically-controlled catheters and/or other percutaneous-access instruments/devices and/or use of such instruments/devices for fluid irrigation and/or aspiration.

In several of the examples described herein, object removal procedures relate to the use of robotic systems and medical instruments/devices used for kidney irrigation and/or aspiration in connection with operations for the removal of stones from a kidney. This disclosure, however, is not limited only to kidney stone treatment. For example, the following description is also applicable to other surgical or medical operations or medical procedures for diagnosis or treatment, such as procedures concerned with the removal of objects from a patient, including any object that can be removed from a treatment site or patient cavity (e.g., the esophagus, ureter, intestine, eye, etc.) or other anatomical site via percutaneous and/or endoscopic access, such as, for example, gallbladder stone removal, lung (pulmonary/transthoracic) tumor biopsy, or cataract removal. Furthermore, although aspects of the present disclosure are described herein for convenience in the context of percutaneous (e.g., nephroscopic) operations, it should be understood that inventive aspects of the present disclosure may be implemented in any suitable or desirable type of medical procedure, whether robotic or not.

With respect to devices and instruments that are used to percutaneously access a treatment site, such as the internal calyx network of a kidney, misalignment of a catheter or other device/instrument with a percutaneous access device/sheath through which access is achieved to the internal anatomy can result in internal tissue damage to the patient and/or damages to device components. Embodiments of the present disclosure relate to alignment devices including an elongate rod or shaft that is configured to be disposed within a lumen of the percutaneous-access device and project/extend from a distal end thereof, wherein such devices may be used during an alignment process during which an alignment path and/or position is determined for a catheter or other instrument relative to the percutaneous-access device. The rod may have a blunt tip portion that reduces the risk of tissue damage caused by sudden sharp movements of the distal tip of the percutaneous-access device/sheath when it is in contact with the internal tissue of the patient. With respect to components of alignment devices disclosed herein, the term "rod" is used according to its broad and ordinary meaning, and may refer to any elongate rod, pole, tube, shaft, cylinder, bar, stick, stem, tube, or the like, whether such structure is hollow or solid in whole or in part.

Alignment devices of the present disclosure further advantageously provide fluid-sealing functionality to create a proximal fluid seal via, for example, a taper-to-taper interaction between a tapered surface or component of the alignment device and corresponding internal tapered surface or component of the percutaneous-access sheath. The term "sheath" is used herein according to its broad and ordinary meaning and may refer to any type of tube, catheter, sleeve, shaft, lumen, conduit, channel, pipe, or other device or structure, or assembly including at least one such device/structure, wherein such device/structure forms or includes at least one lumen or channel through which a device, such as a catheter or other device, can be advanced.

Alignment devices of the present disclosure may further include an alignment structure that is shaped and/or otherwise configured to guide the user to align a component of a robotic instrument device manipulator assembly (referred to herein also as an "instrument manipulator assembly" or a "manipulator assembly" for convenience) associated with a distal portion of a robotic arm with the alignment structure of the alignment device. For example, the alignment structure may be shaped and/or configured to engage/interface with one or more surfaces of an adapter component of the instrument device manipulator assembly. In some embodiments, for example, alignment of the instrument device manipulator assembly and/or component(s) thereof with the alignment structure of the alignment device occurs when the instrument device manipulator assembly is docked on/with the alignment structure of the alignment device and an axially-rotating component of the instrument device manipulator assembly is rotated to align certain alignment features thereof, such as alignment markers or the like, with corresponding markings of the alignment structure of the alignment device. The blunt end of the rod of the alignment device can reduce or eliminate the risk of deleterious effects on the patient in the event that axial motion in the sheath is induced in some manner during the alignment process.

### Medical System

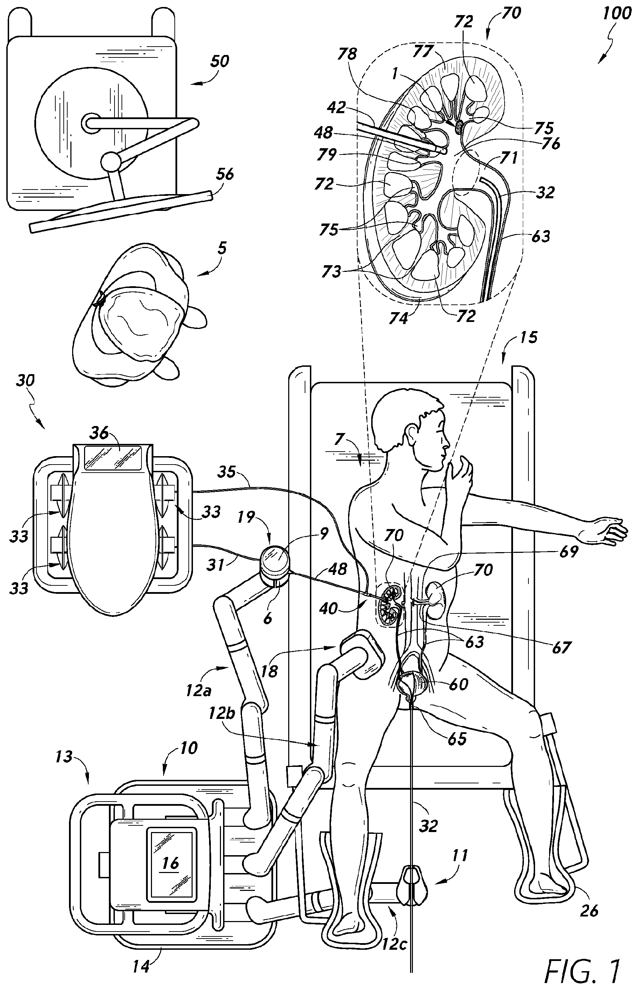

Figure 1 illustrates an example medical system 100 for performing various medical procedures in accordance with aspects of the present disclosure. The medical system 100 may be used for, for example, percutaneous and/or endoscopic (e.g., ureteroscopic) procedures. As referenced and described above, certain ureteroscopic procedures involve the treatment/removal of kidney stones. In some implementations, kidney stone treatment can benefit from the assistance of certain robotic technologies/devices, such as may be similar to those shown in Figure 1 and described in detail below. Robotic medical solutions can provide relatively higher precision, superior control, and/or superior hand-eye coordination with respect to certain instruments compared to strictly-manual procedures. For example, robotic-assisted percutaneous access to the kidney in accordance with some procedures can advantageously enable a urologist to perform both direct-entry endoscopic renal access and percutaneous renal access.

In certain stone management procedures, fluid irrigation may be implemented in order to maintain desired kidney distention, which may advantageously facilitate visualization and/or navigation within the target treatment site (e.g., calyx networks of kidney). Under-pressurization of the kidney or other treatment site can result in the lack of effective anatomical distention for visualization, which can reduce the efficacy of a procedure and/or result in damage to the internal anatomy. Although a human patient 7 is shown, it should be understood that principles disclosed herein are applicable to veterinary procedures as well and animal patients.

Although some embodiments of the present disclosure are presented in the context of nephroscopes (or other examples of percutaneous-access devices), ureteroscopes and/or human renal anatomy, it should be understood that the principles disclosed herein may be implemented in any type of endoscopic and/or percutaneous procedure. Furthermore, several of the examples described herein relate to object removal procedures involving the removal of kidney stones from a kidney. The present disclosure, however, is not limited to kidney stone removal.

The medical system 100 includes a robotic system 10 (e.g., mobile robotic cart) configured to, in some implementations, engage with and/or control a medical instrument 32 (e.g., ureteroscope) to perform a direct-entry procedure on a patient 7. The term "direct-entry" is used herein according to its broad and ordinary meaning and may refer to any entry of instrumentation through a natural or artificial opening in a patient's body. For example, with reference to Figure 1, the direct entry of the scope 32 into the urinary tract of the patient 7 may be made via the urethra 65.

In the illustrated system 100, a percutaneous-access device 40 is implemented to provide percutaneous access to the kidney 70. The percutaneous-access instrument 40 may include one or more sheaths and/or shafts through which instruments and/or fluids may access the target anatomy in which the distal end of the instrument 40 is disposed. The term "percutaneous access" is used herein according to its broad and ordinary meaning and may refer to entry, such as by puncture and/or minor incision, of instrumentation through the skin of a patient and any intervening body layers between the skin and a target anatomical location associated with a procedure (e.g., the calyx network of the kidney 70). The term "percutaneous-access device" is used herein according to its broad and ordinary meaning and may refer to a surgical tool, device, instrument, or assembly that is configured to puncture or to be inserted through skin and/or other tissue/anatomy, such as a needle, a scalpel, a guidewire, sheath, shaft, scope, and the like, and/or an assembly including one or more of the same. However, it should be understood that a percutaneous-access device can refer to other types of medical instruments in the context of the present disclosure. Although described in some contexts herein as a nephroscope, catheter, and an endoscope, respectively, it should be understood that the percutaneous-access instruments 40, 48 and direct-entry instrument 32 may be any type of medical instruments, including endoscopes (such as a ureteroscope), catheters (such as a steerable or non-steerable catheter), nephroscopes, laparoscopes, or other type of medical instrument(s). It should be understood that references herein to percutaneous-access devices can be understood to also relate to other types of access devices, whether or not such access devices pierce, or are configured to pierce, the skin of a patient. For example, such access devices can include access devices configured to access target anatomy via any patient orifice, channel, vessel, pathway, or the like. Urethral access devices, for example, are contemplated within the scope of the embodiments of the present disclosure.

The system 100 may include a catheter 48, which may access the internal renal anatomy through the percutaneous-access device 40. In some embodiments, the catheter 48 may be manipulated and/or held in place by a handle-type coupling 9 coupled to an arm 12a of the robotic system 10, such as an instrument device manipulator assembly/component. The catheter 48 may be a flexible, robotically-driven instrument. In some embodiments, a passive aspiration outflow channel may be formed in the space between the outer wall of the catheter 48 and an inner wall/sheath of the percutaneous-access device/assembly 40, wherein the catheter 48 is disposed within a channel formed by such inner wall/sheath. In some embodiments, active aspiration/suction may be drawn through a lumen of the catheter 48. With the catheter 48 disposed within the percutaneous-access device 40, the catheter 48 and the shaft(s)/sheath(s) of the percutaneous-access device 40 may be generally concentric. The catheter 48 and the percutaneous access device 40 may advantageously have generally circular cross-sectional shapes over at least portions thereof.

The medical system 100 also includes a fluid management cart 30, which may be configured to hold one or more fluid bags/containers 33 and/or control fluid flow thereto/therefrom. For example, an irrigation line 35 may be coupled to one or more of the bags/containers 33 and to an irrigation port of the percutaneous-access device/assembly 40. Irrigation fluid may be provided to the target anatomy via the irrigation line 35 and the percutaneous-access device/assembly 40. The fluid management cart 30 may include certain electronic components, such as a display 36, flow control mechanics, and/or certain associated control circuitry.

The fluid management cart 30 may comprise a stand-alone tower/cart and may have one or more IV bags 33 hanging on one or more sides thereof. The cart 30 may include a pump with which aspiration fluid may be pulled into a collection container/cartridge via an aspiration channel/tube 31. The aspiration channel/tube 31 may be coupled to a catheter handle 9 component of the instrument device manipulator assembly 19.

The medical system 100 also includes a control system 50 configured to interface with the robotic system 10 and/or fluid cart 30, provide information regarding the procedure, and/or perform a variety of other operations. For example, the control system 50 can include one or more display(s) 56 configured to present certain information to assist the physician 5 and/or other technician(s) or individual(s). The medical system 10 can include a table 15 configured to hold the patient 7. The system 10 may further include an electromagnetic (EM) field generator 18, which may be held by one or more of the robotic arms 12 of the robotic system 10 or may be a stand-alone device. Although the various robotic arms are shown in various positions and coupled to various instrumentation, it should be understood that such configurations are shown for convenience and illustration purposes, and such robotic arms may have different configurations over time and/or at different points during a medical procedure. In some embodiments, the arm 12a is configured to hold/control the catheter 48 only after removing the electromagnetic field generator 18 therefrom. That is, the catheter handle 9 and the field generator 18 may generally be mounted to the same robotic arm and interchanged over time.

In some implementations, the system 100 may be used to perform a percutaneous procedure, such as a percutaneous nephrolithotomy (PCNL). To illustrate, if the patient 7 has a kidney stone 1 that is too large to be removed/passed through the urinary tract (63, 60, 65), the physician 5 can perform a procedure to remove the kidney stone 1 through a percutaneous access point/path associated with the flank/side of the patient 7. In some embodiments, the physician 5 can interact with the control system 50 and/or the robotic system 10 to cause/control the robotic system 10 to advance and navigate the medical instrument 32 (e.g., a scope) from the urethra 65, through the bladder 60, up the ureter 63, and into the calyx network of the kidney 70 where the stone 1 is located. The physician 5 can further interact with the control system 50 and/or the robotic system 10 to cause/control the advancement of the catheter 48 through the percutaneous-access instrument 40. The control system 50 can provide information via the display(s) 56 that is associated with the medical instrument 32, such as real-time endoscopic images captured therewith, and/or other instruments of the system 100, to assist the physician 5 in navigating/controlling such instrumentation.

The renal anatomy is described herein for reference with respect to certain medical procedures relating to aspects of the present inventive concepts. The kidneys 70, shown roughly in typical anatomical position in Figure 1, generally comprise two bean-shaped organs located on the left and right sides, respectively, in the retroperitoneal space. In adult humans, the kidneys are generally about 11 cm in height/length. The kidneys receive blood from the paired renal arteries 69; blood exits the kidney via the paired renal veins 67. Each kidney 70 is fluidly coupled with a respective ureter 63, which generally comprises a tube that carries excreted urine from the kidney 70 to the bladder 60.

The kidneys 70 are typically located relatively high in the abdominal cavity and lie in a retroperitoneal position at a slightly oblique angle. The asymmetry within the abdominal cavity, generally caused by the position of the liver, results in the right kidney (shown in detail in Figure 1) typically being slightly lower and smaller than the left, and being placed slightly more to the middle than the left kidney. On top of each kidney is an adrenal gland (not shown). The upper parts of the kidneys 70 are partially protected by the 11th and 12th ribs (not shown). Each kidney, with its adrenal gland, is generally surrounded by two layers of fat: the perirenal fat present between renal fascia and renal capsule and pararenal fat superior to the renal fascia.

The kidneys 70 participate in the control of the volume of various body fluid compartments, fluid osmolality, acid-base balance, various electrolyte concentrations, and removal of toxins. The kidneys 70 provide filtration functionality by secreting certain substances and reabsorbing others. Examples of substances secreted into the urine are hydrogen, ammonium, potassium and uric acid. In addition, the kidneys also carry out various other functions, such as hormone synthesis, and others.

A recessed area on the concave border of the kidney 70 is the renal hilum 78, where the renal artery (not shown in the detailed view of the kidney 70) enters the kidney 70 and the renal vein (not shown in detailed view) and ureter 63 leave. The kidney 70 is surrounded by tough fibrous tissue, the renal capsule 74, which is itself surrounded by perirenal fat, renal fascia, and pararenal fat. The anterior (front) surface of these tissues is the peritoneum, while the posterior (rear) surface is the transversalis fascia.

The functional substance, or parenchyma, of the kidney 70 is divided into two major structures: the outer renal cortex 77 and the inner renal medulla 87. These structures take the shape of a plurality of generally cone-shaped renal lobes, each containing renal cortex surrounding a portion of medulla called a renal pyramid 72. Between the renal pyramids 72 are projections of cortex called renal columns 73. Nephrons (not shown in detail in Figure 1), the urine-producing functional structures of the kidney, span the cortex 77 and medulla 87. The initial filtering portion of a nephron is the renal corpuscle, which is located in the cortex and is followed by a renal tubule that passes from the cortex deep into the medullary pyramids. Part of the renal cortex, a medullary ray, is a collection of renal tubules that drain into a single collecting duct.

The tip/apex, or papilla 79, of each renal pyramid empties urine into a respective minor calyx 75; minor calyces 75 empty into major calyces 76, and major calyces 76 empty into the renal pelvis 71, which transitions to the ureter 63. The manifold-type collection of minor and major calyces may be referred to herein as the "calyx network" of the kidney. At the hilum 78, the ureter 63 and renal vein exit the kidney and the renal artery enters. Hilar fat and lymphatic tissue with lymph nodes surrounds these structures. The hilar fat is contiguous with a fat-filled cavity called the renal sinus. The renal sinus collectively contains the renal pelvis 71 and calyces 75, 76 and separates these structures from the renal medullary tissue. The funnel/tubular-shaped anatomy associated with the calyces can be referred to as the infundibulum/infundibula. That is, an infundibulum generally leads to the termination of a calyx where a papilla is exposed within the calyx.

With further reference to the medical system 100, the medical instrument 32 (e.g., scope, directly-entry instrument, etc.) can be advanced into the kidney 70 through the urinary tract. Once at the site of the kidney stone 1 (e.g., within a target calyx 75 of the kidney 70 through which the stone 1 is accessible), the medical instrument 32 can be used to designate/tag a target location for percutaneous access to the kidney 70. To minimize damage to the kidney and/or surrounding anatomy, the physician 5 can designate a particular papilla 79 of the kidney 70 as the target location / anatomical feature for entering into the kidney 70 with a percutaneous-access instrument (e.g., needle). However, other target locations can be designated or determined. Once the percutaneous-access instrument(s) has/have reached the target location (e.g., calyx 75), the utilized percutaneous access path may be used to extract the kidney stone 1 from the patient 7.

Fluid may advantageously be directed into the calyx network using the fluid cart 30 and irrigation line 35 throughout at least portions of the procedure to produce desirable kidney distension for navigation and viewing. In cases of under-pressurization, wherein there is not enough fluid in the kidney to produce desired or necessary distention, implementation of laser lithotripsy can result in accidental damage to tissue at the treatment site by the laser as a result of the collapse of the surrounding anatomy from under-pressurization.

The various scope-type instruments disclosed herein, such as the scope 32 of the system 100, can be configured to navigate within the human anatomy, such as within a natural orifice or lumen of the human anatomy. The terms "scope" and "endoscope" are used herein according to their broad and ordinary meanings, and may refer to any type of elongate medical instrument having image generating, viewing, and/or capturing functionality and configured to be introduced into any type of organ, cavity, lumen, chamber, or space of a body. A scope can include, for example, a ureteroscope (e.g., for accessing the urinary tract), a laparoscope, a nephroscope (e.g., for accessing the kidneys), a bronchoscope (e.g., for accessing an airway, such as the bronchus), an arthroscope (e.g., for accessing a joint), a cystoscope (e.g., for accessing the bladder), a colonoscope (e.g., for accessing the colon and/or rectum), borescope, and so on. Scopes/endoscopes, in some instances, may comprise a rigid or flexible tube, and may be dimensioned to be passed within an outer sheath, catheter, introducer, or other lumen-type device, or may be used without such devices.

Irrigation fluid may be provided to the treatment site (e.g., kidney 70) through the percutaneous-access device 40, through the percutaneous-access catheter 48, and/or through the direct-entry device 32. Furthermore, irrigation and aspiration may or may not be provided through the same instrument(s). Where one or more of the instruments (32, 40, 48) provides the irrigation and/or aspiration functionality, one or more others of the instruments may be used for other functionality, such as breaking-up the object 80 to be removed.

Once the percutaneous-access device 40 is positioned in the patient, it may be desirable to prevent or reduce the risk of movement of the percutaneous-access device 40. For example, when the percutaneous-access device 40 is initially placed in the patient to provide access to the target treatment site, such placement may be according to the physician's selected position and may be considered an optimal position by the physician. The catheter 48 may generally be advanced through the percutaneous-access device 40 by advancing the instrument device manipulator assembly 19 towards and/or away from the patient 7 to thereby advance the catheter 48 and/or withdraw the catheter 48 from the access device 40. Such movement/advancement of the catheter 48 ideally does not result in undesirable dislodging and/or movement of the percutaneous access device 40, which may cause damage to the patient physiology and/or results in the need to re-set the percutaneous-access device 40 by the physician. Furthermore, improper alignment and/or advancement of the catheter 48 can result in damage to the catheter 48, wherein one or more portions or channels of the catheter along an axial length thereof may become at least partially bent, kinked, broken, torn, dented, and/or otherwise damaged in some manner.

In order to prevent misalignment of the catheter 48 with the access device 40 in a manner as to cause damage to the patient and/or medical instrument(s), it may be necessary or desirable for the catheter 48 (or other instrument) to share the same axis as the percutaneous-access device/sheath 40. Embodiments of the present disclosure, as described in detail below, relate to certain alignment devices that may be utilized to produce and/or facilitate alignment of the catheter 48 or other instrument manipulated by the instrument device manipulator assembly 19 with the axis of the percutaneous-access sheath/device 40. Furthermore, such devices may be configured such that if during the alignment process, the percutaneous-access device 40 is bumped, causing it to move undesirably, the alignment device can prevent or reduce the risk of tissue damage that may otherwise be caused by the relatively thin walls of the percutaneous-access device 40. In addition, with respect to procedures in which instrument alignment is performed while fluid flow is active, such as to create kidney distention, alignment devices of the present disclosure may be configured to seal the percutaneous-access device so that there is substantially no fluid outflow through the percutaneous-access device (e.g., passive outflow) and desired kidney distention is maintained.

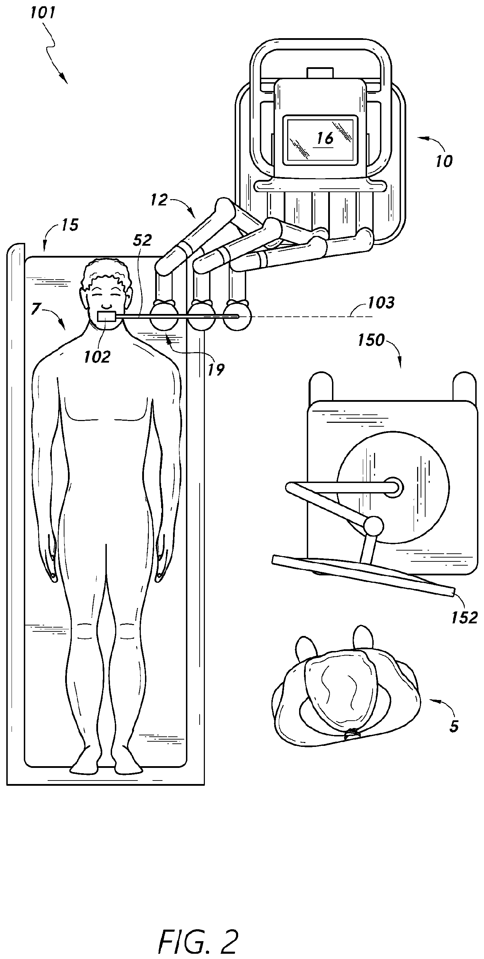

Figure 2 illustrates a cart-based robotic system 101 arranged for diagnostic and/or therapeutic bronchoscopy in accordance with one or more embodiments. During a bronchoscopy, the arm(s) 12 of the robotic system 10 may be configured to deliver a medical instrument, such as a steerable endoscope 52, which may be a procedure-specific bronchoscope for bronchoscopy, to a natural orifice access point (i.e., the mouth of the patient 7 positioned on a table 15 in the present example) to deliver diagnostic and/or therapeutic tools. As shown, the robotic system 10 (e.g., cart) may be positioned proximate to the patient's upper torso in order to provide access to the access point. Similarly, the robotic arms 12 may be actuated to position the bronchoscope 52 relative to the access point. The arrangement in Figure 2 may also be utilized when performing a gastro-intestinal (GI) procedure with a gastroscope, a specialized endoscope for GI procedures.

Once the robotic system 10 is properly positioned, the robotic arms 12 may insert the steerable endoscope 52 into the patient robotically, manually, or a combination thereof. The steerable endoscope 52 may comprise at least two telescoping parts, such as an inner leader portion and an outer sheath portion, each portion coupled to a separate instrument driver from the set of instrument drivers 19, each instrument driver coupled to the distal end of a respective robotic arm 12. This linear arrangement of the instrument drivers 19 creates a "virtual rail" 103 that may be repositioned in space by manipulating the one or more robotic arms 12 into different angles and/or positions. The virtual rails/paths described herein are depicted in the figures using dashed lines that generally do not depict any physical structure of the system. Translation of one or more of the instrument drivers 19 along the virtual rail 103 can advance or retract the endoscope 52 from the patient 7.

The endoscope 52 may be directed down the patient's trachea and lungs after insertion using precise commands from the robotic system 10 until reaching the target operative site. The use of separate instrument drivers 19 can allow independent driving of separate portions of the endoscope/assembly 52. For example, the endoscope 52 may be directed to deliver a biopsy needle to a target, such as, for example, a lesion or nodule within the lungs of a patient. The needle may be deployed down a working channel that runs the length of the endoscope to obtain a tissue sample to be analyzed by a pathologist. Depending on the pathology results, additional tools may be deployed down the working channel of the endoscope for additional biopsies. For example, when a nodule is identified as being malignant, the endoscope 52 may endoscopically deliver tools to resect the potentially cancerous tissue. In some instances, diagnostic and therapeutic treatments can be delivered in separate procedures. In those circumstances, the endoscope 52 may also be used to deliver a fiducial to "mark" the location of the target nodule as well. In other instances, diagnostic and therapeutic treatments may be delivered during the same procedure.

In the system 101, a patient introducer 102 is attached to the patient 7 via a port (not shown; e.g., surgical tube). The patient introducer 102 may be secured to the table 15 (e.g., via a patient introducer holder configured to support the introducer 102 and secure the position of the patient introducer 102 with respect to the table 15 or other structure). In some embodiments, the patient introducer 102 may include a proximal end, a distal end, and an introducer tube therebetween. The proximal end of the patient introducer 102 can provide an opening/orifice which may be configured to receive the surgical instrument 52 (e.g., bronchoscope), and the distal end of the patient introducer 102 can provide a second opening which may be configured to guide the instrument 52 into the patient-access port. A curved tube component of the introducer 102 can connect the proximal and distal ends thereof and guide the instrument 52 through the introducer 102.

The curvature of the introducer 102 may enable the robotic system 10 to manipulate the instrument 52 from a position that is not in direct axial alignment with the patient-access port, thereby allowing for greater flexibility in the placement of the robotic system 10 within the room. Further, the curvature of the introducer 102 may allow the robotic arms 12 of the robotic system 10 to be substantially horizontally aligned with the patient introducer 102, which may facilitate manual movement of the robotic arm(s) 12 if needed.

Alignment devices of the present disclosure may be utilized to provide a solution for aligning the robotic system cart 10 (e.g., an instrument device manipulator assembly 19 associated with one or more of the robotic arms 12) with the patient introducer 102. For example, an alignment device of the present disclosure may be coupled to the patient introducer 102 during an alignment process. In some implementations, the robotic arms 12 of the robotic system 10 may be configured to align along a virtual rail 103 having a major axis that is generally aligned with the opening of the patient introducer 102. Additionally, as with other embodiments disclosed herein, the virtual rail 103 may define a volume in space in which the instrument device manipulator assembl(ies) 19 is/are configured to be arranged during manipulation of the instrument 52 (e.g., during a surgical procedure). Accordingly, alignment of the manipulator assembl(ies) 19 with the patient introducer 102 may improve operational control of the instrument 52 during a surgical procedure.

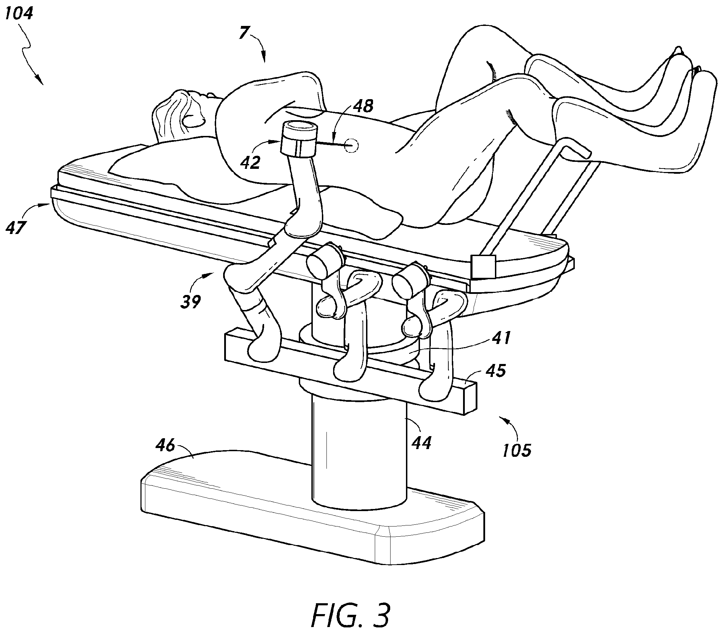

Figure 3 illustrates a table-based robotic system 104 in accordance with one or more embodiments of the present disclosure. By incorporating the robotic components 105 with the table 47, the amount of capital equipment within the operating room may be reduced compared to cart-based robotic systems, which can allow greater access to the patient 7. Much like in the cart-based systems, the end effectors (i.e., instrument device manipulator assemblies) of the robotic arms 39 of the system 104 may generally comprise instrument drivers 42 that are designed to manipulate an elongated medical instrument, such as a catheter 48 or the like, along a virtual rail or path determined during an alignment procedure.

As shown, the robotic-enabled table system 104 can include a column 44 coupled to one or more carriages 41 (e.g., ring-shaped movable structures), from which the one or more robotic arms 39 may emanate. The carriage(s) 41 may translate along a vertical column interface that runs at least a portion of the length of the column 44 to provide different vantage points from which the robotic arms 39 may be positioned to reach the patient 7. The carriage(s) 41 may rotate around the column 44 in some embodiments using a mechanical motor positioned within the column 44 to allow the robotic arms 39 to have access to multiples sides of the table 104. Rotation and/or translation of the carriage(s) 41 can allow the system 104 to align the medical instruments, such as endoscopes and catheters, into different access points on the patient. By providing vertical adjustment, the robotic arms 39 can advantageously be configured to be stowed compactly beneath the platform 47 of the table system 104 and subsequently raised during a procedure.

The robotic arms 39 may be mounted on the carriage(s) 41 through one or more arm mounts 45, which may comprise a series of joints that may individually rotate and/or telescopically extend to provide additional configurability to the robotic arms 39. The column 44 structurally provides support for the table platform 47 and a path for vertical translation of the carriage(s) 41. The column 44 may also convey power and control signals to the carriage(s) 41 and/or the robotic arms 39 mounted thereon.

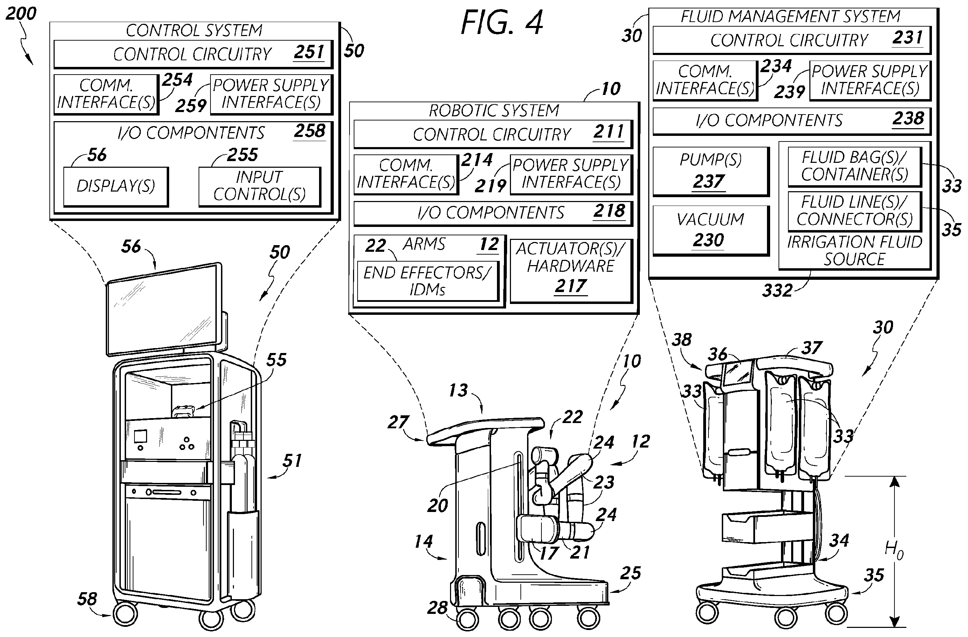

Figure 4 illustrates medical system components that may be implemented in any of the medical systems of Figures 1-3 in accordance with one or more embodiments of the present disclosure. With reference to Figure 4, which shows an example embodiment of the control system 50 of Figures 1 and 2 in accordance with one or more embodiments of the present disclosure, the control system 50 can be configured to provide various functionality to assist in performing a medical procedure. In some embodiments, the control system 50 can be coupled to the robotic system 10 and/or fluid management system 30 and operate in cooperation therewith to perform a medical procedure on the patient 7. For example, the control system 50 can communicate with the robotic system 10 and/or fluid management system 30 via a wireless or wired connection (e.g., to control the robotic system 10, fluid flow from the fluid management system 30, etc.). Further, in some embodiments, the control system 50 can communicate with a needle and/or nephroscope to receive position data therefrom. Moreover, in some embodiments, the control system 50 can communicate with the table 15 to position the table 15 in a particular orientation or otherwise control the table 15. In some embodiments, the control system 50 can communicate with the EM field generator 18 to control generation of an EM field in an area around the patient 7.

Figure 4 further shows an example embodiment of the robotic system 10 of Figures 1 and 2 in accordance with one or more embodiments of the present disclosure. The following description relates to Figures 1, 2, and 4. The robotic system 10 can be configured to at least partly facilitate execution of a medical procedure. The robotic system 10 can be arranged in a variety of ways depending on the particular procedure. The robotic system 10 can include one or more robotic arms 12 configured to engage with and/or control, for example, the scope 32 and/or a percutaneous access catheter 48 to perform one or more aspects of a procedure. As shown, each robotic arm 12 can include multiple arm segments 23 coupled to joints 24, which can provide multiple degrees of movement/freedom. In the example of Figure 1, the robotic system 10 is positioned proximate to the patient's legs and the robotic arms 12 are actuated to engage with and position the scope 32 for access into an access opening, such as the urethra 65 of the patient 7. When the robotic system 10 is properly positioned, the scope 32 can be inserted into the patient 7 robotically using the robotic arms 12, manually by the physician 5, or a combination thereof. An instrument device manipulator assembly including an instrument driver base 6 can be associated with the distal portion of one of the arms 12c to facilitate robotic control/advancement of certain medical instruments, such as the percutaneous-access catheter 48. Any of the percutaneous 40, 48 or direct-entry 32 medical instruments may include one or more working channels through which additional tools, such as lithotripters, basket retrieval devices, forceps, etc., can be introduced into the treatment site.

The robotic system 10 can be coupled to any component of the medical system 100, such as to the control system 50, the table 15, the EM field generator 18, the scope 32, and/or any type of percutaneous-access instrument (e.g., needle, catheter, nephroscope, etc.). In some embodiments, the robotic system 10 is communicatively coupled to the control system 50. For example, the robotic system 10 may be configured to receive a control signal from the control system 50 to perform an operation, such as to position one or more of the robotic arms 12 in a particular manner, manipulate the scope 32, manipulate the catheter 48, and so on. In response, the robotic system 10 can control, using certain control circuitry 211, actuators 217, and/or other components of the robotic system 10, a component of the robotic system 10 to perform the operation. In some embodiments, the robotic system 10 and/or control system 10 is/are configured to receive images and/or image data from the scope 32 representing internal anatomy of the patient 7, namely the urinary system with respect to the particular depiction of Figure 1, and/or display images based thereon.

With reference to Figure 4, the robotic system 10 generally includes an elongated support structure 14 (also referred to as a "column"), a robotic system base 25, and a console 13 at the top of the column 14. The column 14 may include one or more arm supports 17 (also referred to as a "carriage") for supporting the deployment of the one or more robotic arms 12 (three shown in Figures 1 and 2). The arm support 17 may include individually-configurable arm mounts that rotate along a perpendicular axis to adjust the base of the robotic arms 12 for better positioning relative to the patient.

The arm support 17 can be configured to vertically translate along the column 14. For example, the arm support 17 can be connected to the column 14 through slots 20 that are positioned on opposite sides of the column 14 to guide the vertical translation of the arm support 17. The slots 20 contain a vertical translation interface to position and hold the arm support 17 at various vertical heights relative to the robotic system base 25. Vertical translation of the arm support 17 allows the robotic system 10 to adjust the reach of the robotic arms 12 to meet a variety of table heights, patient sizes, and physician preferences. Similarly, the individually-configurable arm mounts on the arm support 17 can allow the robotic arm base 21 to be angled in a variety of configurations.

The robotic arms 12 may generally comprise robotic arm bases 21 and end effectors 22 (e.g., instrument drivers), separated by a series of linkages 23 that are connected by a series of joints 24, each joint comprising one or more independent actuators 217. Each actuator may comprise an independently-controllable motor. Each independently-controllable joint 24 can provide or represent an independent degree of freedom available to the robotic arm. **In** some embodiments, each of the arms 12 has seven joints, and thus provides seven degrees of freedom, including "redundant" degrees of freedom. Redundant degrees of freedom allow the robotic arms 12 to position their respective end effectors 22 at a specific position, orientation, and trajectory in space using different linkage positions and joint angles. This allows for the system to position and direct a medical instrument from a desired point in space while allowing the physician to move the arm joints into a clinically advantageous position away from the patient to create greater access, while avoiding arm collisions.

The robotic system base 25 balances the weight of the column 14, arm support 17, and arms 12 over the floor. Accordingly, the robotic system base 25 may house certain relatively heavier components, such as electronics, motors, power supply, as well as components that selectively enable movement or immobilize the robotic system. For example, the robotic system base 25 can include wheel-shaped casters 28 that allow for the robotic system to easily move around the operating room prior to a procedure. After reaching the appropriate position, the casters 28 may be immobilized using wheel locks to hold the robotic system 10 in place during the procedure.

Positioned at the upper end of column 14, the console 13 can provide both a user interface for receiving user input and a display screen 16 (or a dual-purpose device such as, for example, a touchscreen) to provide the physician/user with pre-operative and/or intra-operative data. Potential pre-operative data on the console/display 16 or display 56 may include pre-operative plans, navigation and mapping data derived from pre-operative computerized tomography (CT) scans, and/or notes from pre-operative patient interviews. Intra-operative data on display may include optical information provided from the tool, sensor and coordinate information from sensors, as well as vital patient statistics, such as respiration, heart rate, and/or pulse. The console 13 may be positioned and tilted to allow a physician to access the console from the side of the column 14 opposite the arm support 17. From this position, the physician may view the console 13, robotic arms 12, and patient while operating the console 13 from behind the robotic system 10. As shown, the console 13 can also include a handle 27 to assist with maneuvering and stabilizing robotic system 10.

The end effector 22 of each of the robotic arms 12 may serve as a driver base of an instrument device manipulator assembly, which can include an instrument handle, which may be attached using an adapter. In some embodiments, the instrument handle can be removed and replaced with a different type of instrument, for example, a first type of instrument handle may manipulate an endoscope, while a second type of instrument handle may manipulate a catheter. Another type of instrument handle may be configured to hold an electromagnetic field generator 18. An adapter can include connectors to transfer pneumatic pressure, electrical power, electrical signals, and/or optical signals from the robotic arm 12 to the instrument handle. The instrument handles may be configured to manipulate medical instruments (e.g., surgical tools/instruments), such as the catheter 48, using techniques including, for example, direct drives, harmonic drives, geared drives, belts and pulleys, magnetic drives, and the like. In some embodiments, the instrument manipulators 22 can be associated with respective ones of the robotic arms 212, wherein the robotic arms 212 are configured to insert or retract the respective coupled medical instruments into or out of the treatment site (e.g., through a percutaneous-access sheath/device).

As referenced above, the systems of Figures 1, 2, and 3 can include certain control circuitry configured to perform certain of the functionality described herein, including the control circuitry 211 of the robotic system 10, the control circuitry 231 of the fluid management system 230, and/or the control circuitry 251 of the control system 50. That is, the control circuitry of the systems of Figures 1 and 2 may be part of the robotic system 10, the fluid management system 30, the control system 50, or some combination thereof. Therefore, any reference herein to control circuitry may refer to circuitry embodied in a robotic system, a fluid management system, a control system, or any other component of a medical system. The term "control circuitry" is used herein according to its broad and ordinary meaning, and may refer to any collection of processors, processing circuitry, processing modules/units, chips, dies (e.g., semiconductor dies including come or more active and/or passive devices and/or connectivity circuitry), microprocessors, micro-controllers, digital signal processors, microcomputers, central processing units, field programmable gate arrays, programmable logic devices, state machines (e.g., hardware state machines), logic circuitry, analog circuitry, digital circuitry, and/or any device that manipulates signals (analog and/or digital) based on hard coding of the circuitry and/or operational instructions. Control circuitry referenced herein may further include one or more circuit substrates (e.g., printed circuit boards), conductive traces and vias, and/or mounting pads, connectors, and/or components. Control circuitry referenced herein may further comprise one or more, storage devices, which may be embodied in a single memory device, a plurality of memory devices, and/or embedded circuitry of a device. Such data storage may comprise read-only memory, random access memory, volatile memory, non-volatile memory, static memory, dynamic memory, flash memory, cache memory, data storage registers, and/or any device that stores digital information. It should be noted that in embodiments in which control circuitry comprises a hardware and/or software state machine, analog circuitry, digital circuitry, and/or logic circuitry, data storage device(s)/register(s) storing any associated operational instructions may be embedded within, or external to, the circuitry comprising the state machine, analog circuitry, digital circuitry, and/or logic circuitry.

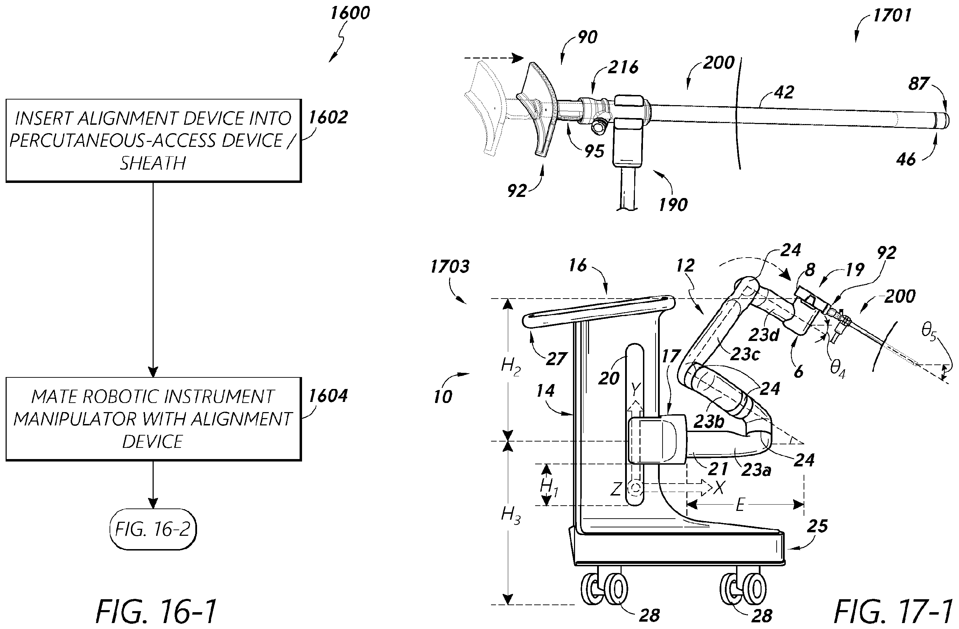

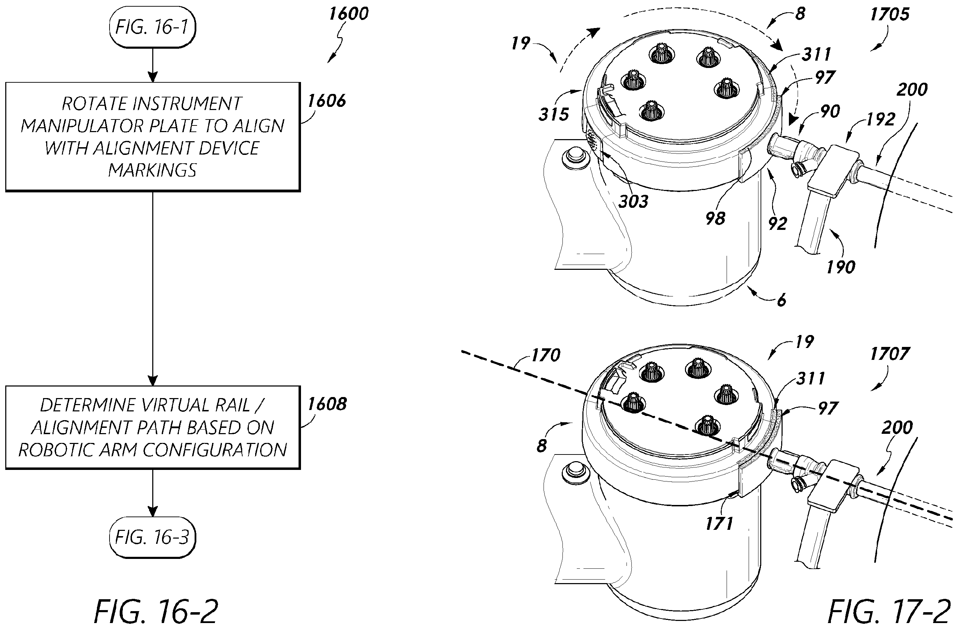

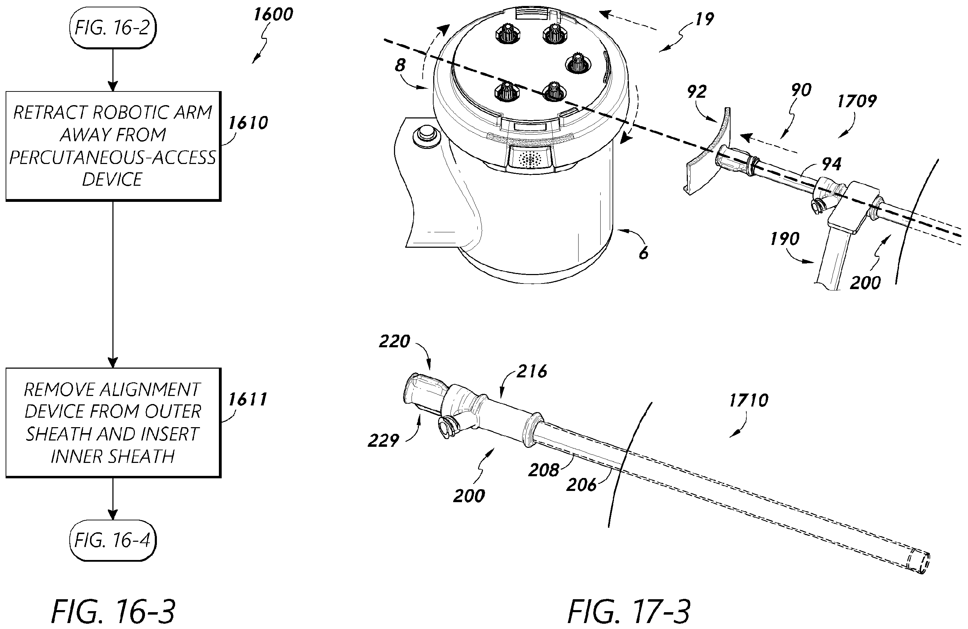

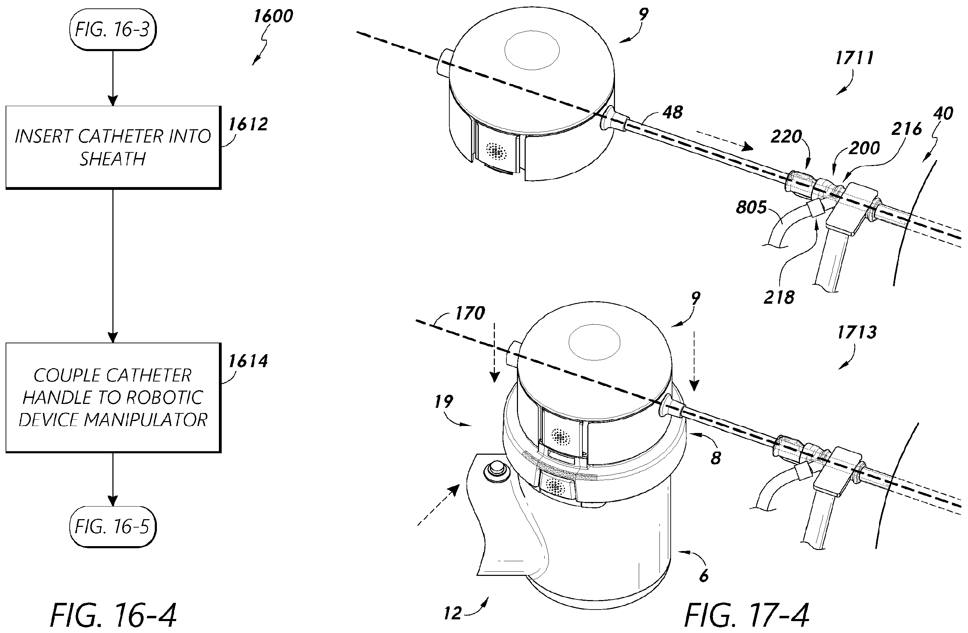

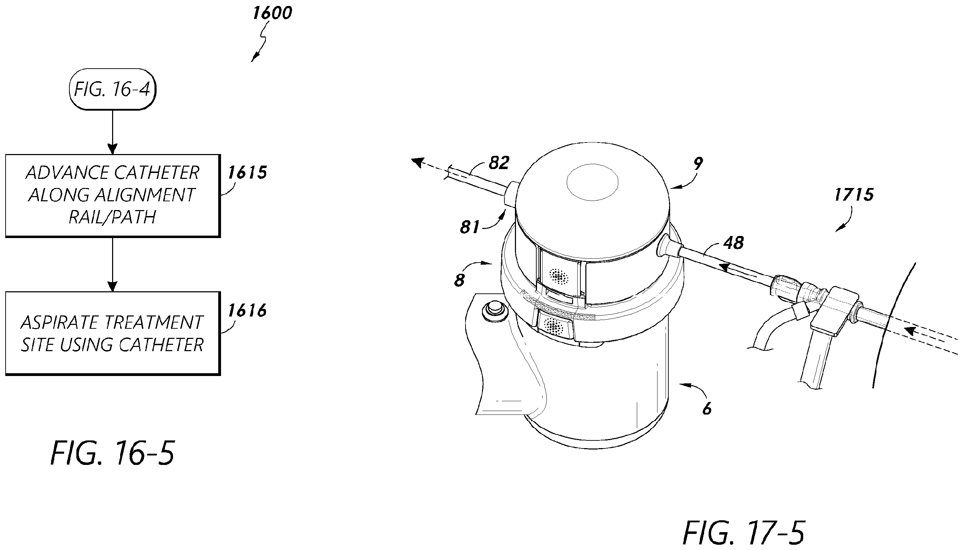

The control circuitry 211, 231, and/or 251 may comprise a computer-readable medium storing, and/or configured to store, hard-coded and/or operational instructions corresponding to at least some of the steps and/or functions illustrated in one or more of the present figures and/or described herein. Such computer-readable medium can be included in an article of manufacture in some instances. The control circuitry 211/231/251 may be entirely locally maintained/disposed or may be remotely located at least in part (e.g., communicatively coupled indirectly via a local area network and/or a wide area network). Any of the control circuitry 211, 231, 251 may be configured to perform any aspect(s) of the various processes disclosed herein, including the processes shown in Figures 16 and 17 and described below.

With respect to the robotic system 10, at least a portion of the control circuitry 211 may be integrated with the base 25, column 14, and/or console 13 of the robotic system 10, and/or another system communicatively coupled to the robotic system 10. With respect to the fluid management system 30, at least a portion of the control circuitry 231 may be integrated with the base 35, column 34, and/or console 38 of the fluid management system 30, and/or another system communicatively coupled to the fluid management system 30. With respect to the control system 50, at least a portion of the control circuitry 251 may be integrated with the console base 51 and/or display unit 56 of the control system 50. It should be understood that any description herein of functional control circuitry or associated functionality may be understood to be embodied in the robotic system 10, the fluid management system 30, the control system 50, or any combination thereof, and/or at least in part in one or more other local or remote systems/devices.

With further reference to Figure 4, the control system 50 can include various I/O components 258 configured to assist the physician 5 or others in performing a medical procedure. For example, the input/output (I/O) components 258 can be configured to allow for user input to control/navigate the scope 32 and/or catheter 48 within the patient 7. In some embodiments, for example, the physician 5 can provide input to the control system 50 and/or robotic system 10, wherein in response to such input, control signals can be sent to the robotic system 10 to manipulate the scope 32 and/or catheter 48. The control system 50 can include one or more display devices 56 to provide various information regarding a procedure. For example, the display(s) 56 can provide information regarding the scope 32 and/or catheter 48. For example, the control system 50 can receive real-time images that are captured by the scope 32 and display the real-time images via the display(s) 56. Additionally or alternatively, the control system 50 can receive signals (e.g., analog, digital, electrical, acoustic/sonic, pneumatic, tactile, hydraulic, etc.) from a medical monitor and/or a sensor associated with the patient 7, and the display(s) 56 can present information regarding the health or environment of the patient 7. Such information can include information that is displayed via a medical monitor including, for example, information relating to heart rate (e.g., ECG, HRV, etc.), blood pressure/rate, muscle bio-signals (e.g., EMG), body temperature, blood oxygen saturation (e.g., SpO₂), CO₂, brainwaves (e.g., EEG), environmental and/or local or core body temperature, and so on.

To facilitate the functionality of the control system 50, the control system 50 can include various components (sometimes referred to as "subsystems"). For example, the control system 50 can include the control electronics/circuitry 251, as well as one or more power supplies/supply interfaces 259, pneumatic devices, optical sources, actuators, data storage devices, and/or communication interfaces 254. In some embodiments, the control system 50 is movable, while in other embodiments, the control system 50 is a substantially stationary system. Although various functionality and components are discussed as being implemented by the control system 50, any of such functionality and/or components can be integrated into and/or performed by other systems and/or devices, such as the robotic system 10, the fluid management system 30, the table 15, and/or others, for example.

With further reference to Figures 1 and 2, the medical systems 100/101 can provide a variety of benefits, such as providing guidance to assist a physician in performing a procedure (e.g., fluid management status/conditions, instrument tracking, instrument alignment information, etc.), enabling a physician to perform a procedure from an ergonomic position without the need for awkward arm motions and/or positions, enabling a single physician to perform a procedure with one or more medical instruments, avoiding radiation exposure (e.g., associated with fluoroscopy techniques), enabling a procedure to be performed in a single operative setting, providing continuous suction to remove an object more efficiently (e.g., to remove a kidney stone), and so on. For example, the medical systems 100/101 can provide guidance information to assist a physician in using various medical instruments to access a target anatomical feature while minimizing bleeding and/or damage to anatomy (e.g., critical organs, blood vessels, etc.). Further, the medical systems 100/101 can provide non-radiation-based navigational and/or localization techniques to reduce physician and patient exposure to radiation and/or reduce the amount of equipment in the operating room. Moreover, the medical systems 100/101 can provide functionality that is distributed between two or more of the control system 50, the fluid management system 30, and the robotic system 10, which may be independently movable. Such distribution of functionality and/or mobility can enable the control system 50, the fluid management system 30, and/or the robotic system 10 to be placed at locations that are optimal for a particular medical procedure, which can maximize working area around the patient 7 and/or provide an optimized location for the physician 5 to perform a procedure.

The various components of the systems 100/101 can be communicatively coupled to each other over a network, which can include a wireless and/or wired network. Example networks include one or more personal area networks (PANs), local area networks (LANs), wide area networks (WANs), Internet area networks (IANs), cellular networks, the Internet, personal area networks (PANs), body area network (BANs), etc. For example, the various communication interfaces of the systems of Figure 2 can be configured to communicate with one or more device/sensors/systems, such as over a wireless and/or wired network connection. In some embodiments, the various communication interfaces can implement a wireless technology such as Bluetooth, Wi-Fi, near field communication (NFC), or the like. Furthermore, in some embodiments, the various components of the system 10 can be connected for data communication, fluid exchange, power exchange, and so on via one or more support cables, tubes, or the like.

The control system 50, fluid management, and/or robotic system 10 can include certain user controls 255 (e.g., controls 55), which may comprise any type of user input (and/or output) devices or device interfaces, such as one or more buttons, keys, joysticks, handheld controllers (e.g., video-game-type controllers), computer mice, trackpads, trackballs, control pads, and/or sensors (e.g., motion sensors or cameras) that capture hand gestures and finger gestures, touchscreens, and/or interfaces/connectors therefore. Such user controls are communicatively and/or physically coupled to respective control circuitry.

In some embodiments, a user can manually manipulate a robotic arm 12 of the robotic system 10 without using electronic user controls. For example, during setup in a surgical operating room, a user may manually move the robotic arms 12 and/or any other medical instruments to provide desired access to a patient 7. The robotic system 10 may rely on force feedback and inertia control from the user to determine appropriate configuration of the robotic arms 12 and associated instrumentation.

The fluid management system 30 and/or control system 50 can include one or more pumps, flow meters, valve controls, and/or other fluid-/flow-control components (e.g., sensor devices, such as pressure sensors) in order to provide controlled irrigation and/or aspiration/suction capabilities for a medical instrument. In some embodiments, irrigation and aspiration capabilities can be delivered directly to/from a medical instrument through separate cable(s). In some embodiments, the control system 50 can be coupled to the robotic system 10, the table 15, and/or a medical instrument, such as the percutaneous-access device 40 and/or a needle or other percutaneous-access instrument (not shown), through one or more cables or connections (not shown). In some embodiments, the control circuitry 251 of the control system 50 (or the control circuitry 231 fluid management system 30 or the control circuitry 211 of the robotic system to 10) can generate and provide one or more signals to the fluid management system 30 to indicate how high (or low) the irrigation pressure level from the irrigation fluid source 332 can go, wherein such signals can be translated by the fluid management system 30 into irrigation outputs with respect to operation of the pump(s) 237 and/or other flow-control device(s) associated with the irrigation fluid source 332.

Any of the control circuitry 231, the control circuitry 251, and the control circuitry 211 may be configured to control the pump(s) 237 and/or the vacuum 238 to provide irrigation pressure limitation in accordance with aspects of the present disclosure. Any of the medical instruments 32, 40, 48 shown in the system 100 may be attached to the pump(s) 237, which may facilitate irrigation fluid flow. Although Figure 4 includes pump(s) 238, in some embodiments, irrigation fluid flow is achieved without the use of pumps, wherein such flow is driven primarily by gravitational potential force. The pump(s) 237 is/are attached to the irrigation source 332, which provides irrigation fluid (e.g., saline solution) to be pumped through one or more of the medical instruments 32, 40, 48 and into the treatment site. In some examples, the pump(s) 237 is/are peristaltic pump(s). In some embodiments, the pump(s) 237 can be replaced with a vacuum that is configured to apply a vacuum pressure to draw the irrigation fluid from the irrigation fluid source 332 and out through the respective coupled medical instrument.

One or more of the percutaneous-access and/or direct-entry instruments implemented in connection with the systems 100, 200 may be fluidly coupled/connected to a vacuum 238 configured to facilitate fluid aspiration. For example, the vacuum 238 can be configured to apply a negative pressure to draw fluid out of the treatment site. The vacuum 238 may be connected to a collection container into which withdrawn fluid is collected. In some examples, aspiration suction may be facilitated by one or more pumps rather than a vacuum. Furthermore, in some embodiments, aspiration is primarily passive, rather than through active suction. Therefore, it should be understood that embodiments of the present disclosure may not include vacuum components.

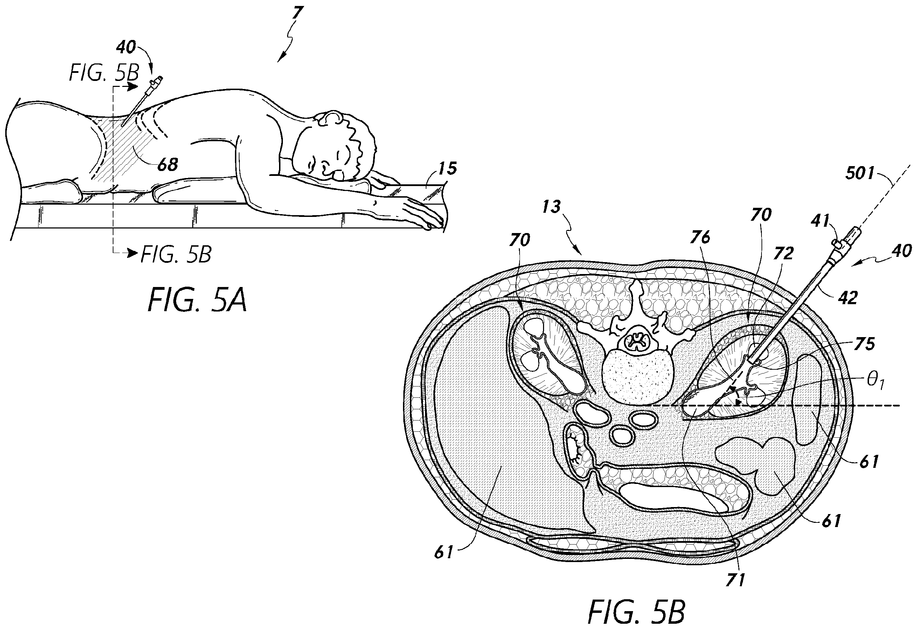

Figures 5A and 5B show side and cross-sectional views, respectively, of a patient 7 in a prone position having a percutaneous-access device 40 (e.g., sheath assembly) disposed at least partially in his/her flank 68 and/or renal anatomy in accordance with one or more embodiments. As apparent in Figure 5B, the prone position may be desirable for percutaneous access to posterior calyces of kidneys due to the position of the flank 68 of the patient and the access path relative to the position/presence of other critical anatomy 61.

For a PCNL procedure, as shown in Figures 5A and 5B, percutaneous access to a posterior calyx 75 of a kidney 70 may result in a percutaneous-access device 40 having a sheath angle of around 45° above the horizon. Therefore, in order to achieve alignment of a catheter or other robotically-controlled instrument with the access device 40, the device manipulator assembly associated with the catheter/instrument may be oriented at the angle *θ₁* above the horizon. For example, the catheter may be advanced at least in part along the virtual rail 501 corresponding to the axis of the sheath 42.

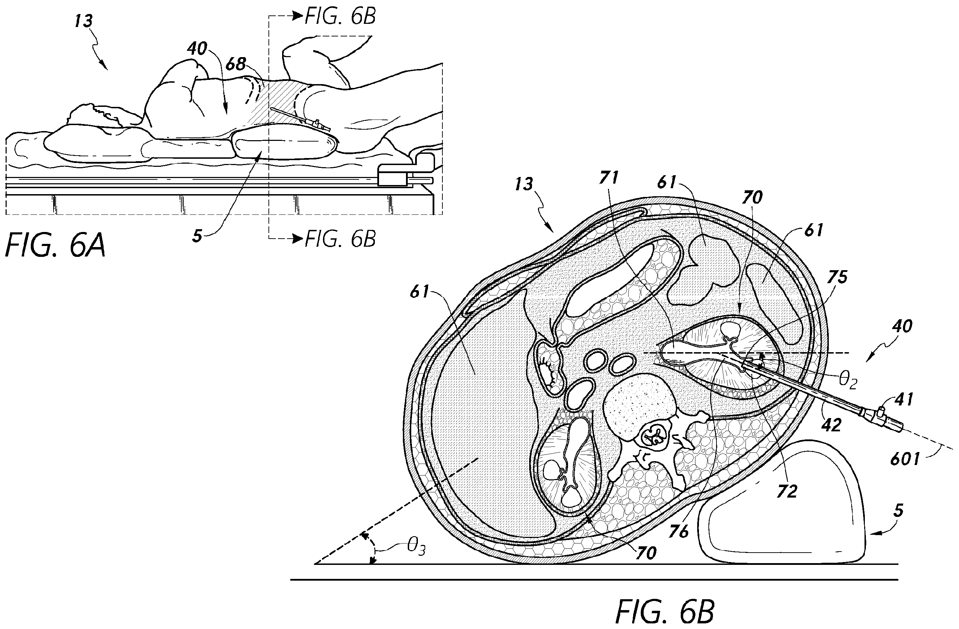

Figures 6A and 6B show side and cross-sectional views, respectively, of a patient in a modified supine position having a percutaneous-access device 40 disposed at least partially in his/her flank 68 and/or renal anatomy in accordance with one or more embodiments. In the modified-supine position, the sheath angle *θ₂* may generally be somewhat closer to the horizon than with respect to certain prone procedures due to the orientation of the posterior calyces being generally downward, wherein such downward trajectory is offset at least in part by the tilting of the patient 7 by some angle *θ₃* to provide greater exposure for the flank 68 of the patient (e.g., using a pillow or other structure 5 to wedge/support the patient).

As shown in Figures 6A and 6B, for percutaneous PCNL procedures in the modified supine position, percutaneous access to a posterior calyx 75 of a kidney 70 may result in a percutaneous-access device 40 having a sheath angle below the horizon, as shown as *θ₂* in Figure 6B. Generally, in a modified supine position, the patient 7 may be propped up using a pillow 5 or similar apparatus to raise the flank 68 of the patients on one side. The prop angle *θ₃* may generally be an angle between 0°-45° above the horizon, as shown. Therefore, in order to achieve alignment of a catheter or other robotically-controlled instrument with the access device 40, the device manipulator assembly associated with the catheter/instrument may be oriented at the angle *θ₂* below the horizon. For example, the catheter may be advanced at least in part along the virtual rail 601 corresponding to the axis of the sheath 42.

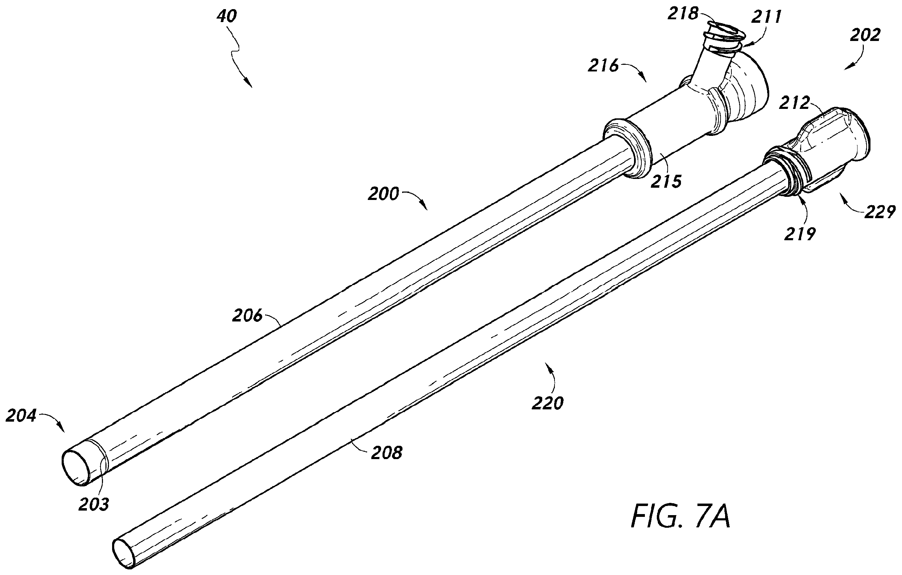

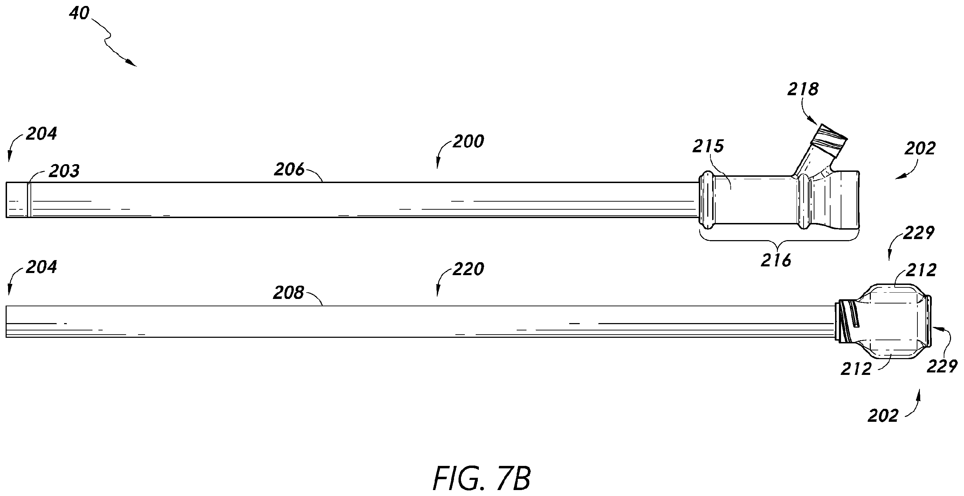

Figures 7A and 7B show isomeric and side views, respectively, of inner 220 and outer 200 sheath devices/components of a percutaneous-access device/assembly 40 in accordance with one or more embodiments. In the illustrated embodiment, the sheath components extend between proximal 202 and distal 204 ends. The distal end 204 of the outer sheath component 200 can be configured to be inserted into a treatment site of a patient with or without the inner sheath component 220 being assembled therewith.

In an assembled configuration, the inner sheath component 220 may be secured at least partially within the outer sheath component 200, wherein a hub 229 of the inner sheath component 220 is screwed into or otherwise secured to the hub 216 of the outer sheath component 200, such that the shaft 208 of the inner sheath component 220 is disposed within and coaxial with the shaft 206 of the outer sheath component 200. In some embodiments, the distal end 204 of the outer shaft 206 is sharpened, tapered, angled, or otherwise configured so that it can create a percutaneous cut in the patient through which the outer sheath component 200 can be inserted. In some embodiments, the distal end 204 is inserted through a previously formed percutaneous cut, opening, or port.

In general, during use of the percutaneous-access device 40, the distal end 204 is positioned within the treatment site and the proximal end 202 is positioned outside of the patient's body. As will be described below, the percutaneous-access device 40 can provide one or more paths, channels, or conduits from the proximal end 202 to the distal end 204 through which medical instruments can be inserted to reach the treatment site. The paths, channels, and conduits can also be used to provide irrigation and aspiration of fluid through the percutaneous access sheath 200. When assembled, the outer 206 and inner 208 sheaths/conduits of the percutaneous-access device 40 are configured such that the distal end 204 of the inner shaft 208 is flush with or positioned proximally of the distal end 204 of the outer shaft 206.

In some embodiments, the inner sheath component 220 can be removable. For example, the inner shaft 208 can be configured to slide out of the outer shaft 206. In some embodiments, when the inner shaft 208 is positioned within the outer shaft 206, it can be secured by a valve (e.g., a rubber valve or O-ring) to provide a seal between the proximal ends 202 of the outer sheath component 200 and the inner sheath component 220. In some embodiments, the inner sheath component 220 can be removable from the outer sheath component 200 to allow a dilator to fit through the outer shaft 206. In some embodiments, the inner sheath component 220 is fixed (e.g., permanently fixed) within the outer sheath component 200 and cannot be removed.

The outer shaft 206 can comprise a type of tube, conduit, or pipe forming a lumen. In some embodiments, the outer shaft 206 is rigid. For example, the outer shaft 206 can be sufficiently rigid such that it can be percutaneously inserted into the patient. In some embodiments, the outer shaft 206 comprises a hypotube. Use of a hypotube can provide relatively thin sidewalls so that the overall the dimensions/profile of the percutaneous-access device 40 can be minimized while maintaining sufficient strength and rigidity. This can reduce the tract size of the percutaneous-access device 40, which can reduce potential patient complications. In some embodiments, the outer shaft 206 comprises stainless steel, although other suitable materials can also be used. In the illustrated embodiment, the outer shaft 206 has a substantially circular cross-section over at least a portion of the length thereof. The outer shaft 206 can have an outer diameter of 23 Fr. (0.302 inches) and an inner diameter of 21.4 Fr. (0.281 inches). These dimensions are provided by way of example, and other dimensions for the outer shaft 206 are possible. For example, the outer diameter of the outer shaft 206 can be about 21 Fr., about 22 Fr., about 23 Fr., about 24 Fr., or about 25 Fr., as well as other sizes both larger and smaller. Additionally, the inner diameter of the outer shaft 206 can be about 18 Fr. About 19 Fr., about 20 Fr., about 21 Fr., about 22 Fr., or about 23 Fr., as well as other sizes both larger and smaller. (1 Fr. = 1/3 mm).

The hub 216 of the outer sheath component 200 can be positioned at the proximal end 202 of the outer shaft 206. In some embodiments, the outer shaft 206 is welded to the hub 216, although other methods for joining the outer shaft 206 and the hub 216 are possible. A fluid inlet 218 can be positioned on the hub 216. As will be described in more detail below, the fluid inlet 218 can be configured to connect to an irrigation source such that irrigation can be provided through the percutaneous-access device 40 and into the treatment site. In the illustrated embodiment, the fluid inlet 218 is configured as a side port. For example, a longitudinal axis of the fluid inlet can be transverse (e.g., angled or perpendicular) to a longitudinal axis of the outer shaft 206. In the illustrated embodiment, the fluid inlet 218 is configured as a Luer connector. Other types of connectors can be used in other embodiments. Further, in some embodiments, the fluid inlet 218 can be positioned in other locations on the hub 216 and/or other portions of the outer sheath component 200.

In some embodiments, the hub 216 is made from extruded plastic, although other methods of manufacture and types of materials are also possible. In some embodiments, the hub 216 comprises plastic (such as ABS plastic, polycarbonate, or other suitable plastics) overmolded onto the outer shaft 206. In some embodiments, the percutaneous-access device 40 can also include a hub 229 on the inner shaft 208. The hub 229 of the inner sheath component 220 can facilitate engagement and sealing between the outer sheath component 200 and the inner sheath component 220. In some embodiments, the hub 229 of the inner sheath component 220 comprises plastic (such as ABS plastic, polycarbonate, or other suitable plastics) overmolded onto the inner shaft 208.

The hub 216 of the outer sheath component 200 may also include a valve positioned therein. For example, when the inner shaft 208 is positioned within the outer shaft 206, the inner shaft 208 may extend through the valve of the hub 216. The valve of the hub 216 may seal the proximal end 202 of the outer shaft 206.

The inner shaft 208 can comprise a type of conduit, tube, or pipe. In some embodiments, the inner shaft 208 is rigid. For example, the inner shaft 208 can be sufficiently rigid such that it can be percutaneously inserted into the patient. In some embodiments, the inner shaft 208 comprises a hypotube. In some embodiments, the inner shaft 208 comprises stainless steel, although other materials can also be used. The inner shaft 208 can have a substantially circular cross-section over at least a portion of its length. The inner shaft 208 can have an outer diameter of 19.1 Fr. (0.251 inches) and an inner diameter of 18.1 Fr. (0.238 inches). In some embodiments, the inner shaft 208 has a diameter of about 16 Fr., 17 Fr., 18 Fr., 19 Fr., 20 Fr., 21 Fr., or 22 Fr., or other size, whether larger or smaller. Additionally, the inner diameter of the inner shaft 208 can be about 14 Fr., 15 Fr., 16 Fr., 17 Fr., 18 Fr., 19 Fr., or 20 Fr., or other size, whether larger or smaller. (1 Fr. = 1/3 mm).

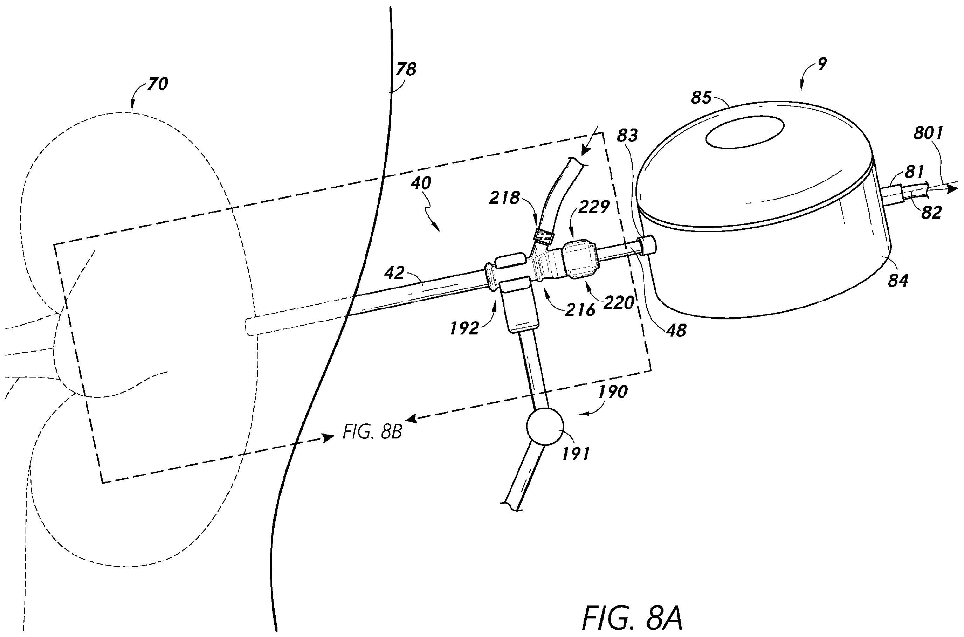

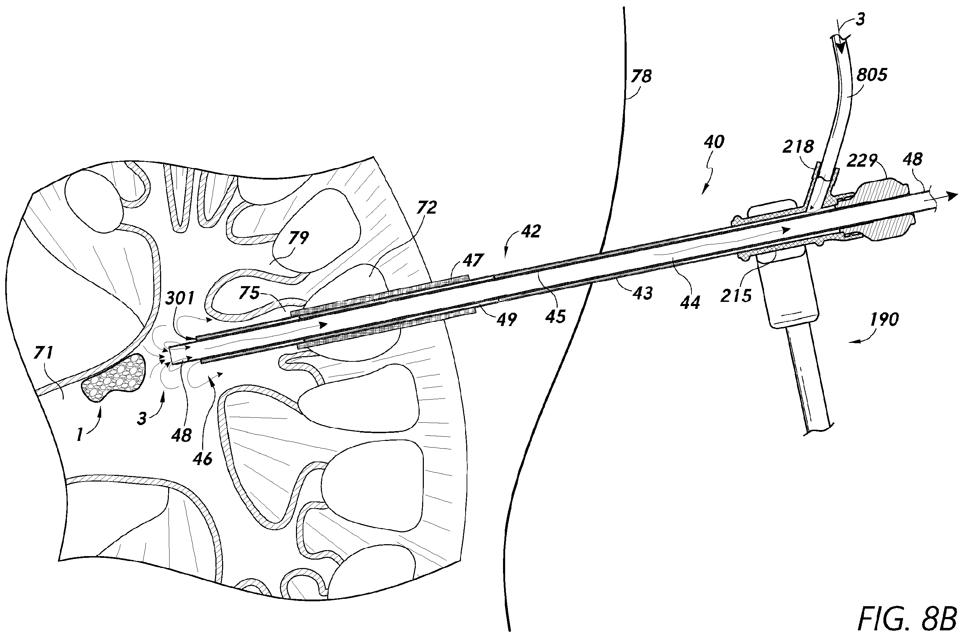

Figures 8A and 8B show perspective and side cut-away close-up views, respectively of a percutaneous-access system disposed in portions of the renal anatomy of a patient in accordance with one or more embodiments. The system of Figures 8A and 8B includes a percutaneous-access device 40 and a robotically-controlled catheter 48 aligned with an axis 801 of a sheath 42 thereof, wherein the catheter 48 is disposed at least partially within a lumen of the access device 40.

As referenced above, various aspects of the present disclosure relate to systems, devices, and methods for aligning robotically-controlled catheters and/or other instruments with a percutaneous-access device. Such catheters/instruments may be used for managing aspiration fluid flow for a treatment site (e.g., kidney). Fluid irrigation and aspiration, which may be referred to as "fluidics" herein, can represent an important component of certain medical procedures, such as percutaneous nephrolithotomy (PCNL). For example, during PCNL, fluidics may be applied to clear stone dust, small fragments, and thrombus from the treatment site as well as the visual field provided by the medical instrument(s). For example, with respect to the embodiment of Figures 8A and 8B, irrigation fluid 3 can be provided through a channel/port 218 of the percutaneous-access instrument 40. Aspiration (outflow) may exit the treatment site through one or more passive and/or active outflow channels, such as through the catheter 48, instrument handle 9, and/or port 81. **In** some embodiments, irrigation and aspiration can both be active.

The diagram of Figures 8A and 8B may be representative of a PCNL procedure. The illustrated renal anatomy includes an object 1 disposed in the calyx network of the kidney 70, wherein the object 1 can be any object that is targeted for removal, such as a kidney stone. The percutaneous-access device 40 can be inserted percutaneously into the kidney 70 through a flexible sheath 47. According to some implementations, the flexible sheath 47 may be placed by first accessing the treatment site with a rigid needle and using a dilator to dilate the percutaneous-access path and place the sheath 47. The percutaneous-access device 40 can include a working channel 44 within an inner shaft/wall 45, though which various tools can be inserted, such as the catheter 48. In some implementations, a lithotripter (such as an ultrasonic lithotripter) may be inserted through the working channel 44 of the percutaneous-access device 40. The percutaneous-access device 40 can also include an optic device (not shown) configured to allow a surgeon to visualize the treatment site.

The catheter 48 may be navigated within the kidney 70 by torqueing the catheter 48 towards the object 1 using certain actuators/mechanisms associated with the catheter handle 9. In some implementations, the object/stone 80 may be broken-up using a lithotripter (not shown) and removed in smaller fragments through the percutaneous-access catheter 48. The lithotripter may be advanced to the treatment site through percutaneous or direct entry.

As illustrated with arrows in Figure 8B, irrigation (e.g., saline solution) can be applied to the treatment site (e.g., the kidney 70) through the percutaneous-access instrument 40. The irrigation fluid 3 may enter the percutaneous-access device 40 through an irrigation port 218 and exit through a distal end 301 into the kidney 70. Irrigation can be used to clear stone dust and small fragments from the field of view of a scope or other imaging/viewing device to allow the surgeon to visualize the treatment site, as well as to distend the kidney 70 to allow access to the object 1. In the illustrated example, aspiration is also applied from the treatment site through the medical device 40. As shown, fluid can be passively removed from the kidney 70 through the working channel 44 of the percutaneous-access device (e.g., between the inner shaft 45 of the percutaneous-access device 40 and the catheter 48). In some instances, aspiration is pulled (actively) through one or more outflow channels and/or permitted to passively flow through one or more outflow channels. For example, active aspiration/suction may be drawn through the catheter 48. In some implementations, fluidics are applied during substantially the entire procedure. The hub 229 of the inner sheath component 220 can include certain fluid-sealing features configured to prevent or reduce fluid leakage to thereby allow for maintenance of desired fluid conditions within the target anatomy.

The fluidics applied during the procedure can establish a fluid flow as illustrated by the arrows in Figure 8B. Initially, fluid can flow outward from the distal tip 301 of the percutaneous-access device 40 towards the object 1. Aspiration through the catheter 48 can cause fluid flow back towards the percutaneous-access device/sheath 40. As illustrated, in the region of the object 1, the flow may be both directed toward and away from the object 1 with respect to the distal end 301 of the percutaneous-access device 40. In the absence of sufficient fluid aspiration, risks of over-filling the kidney can be present.

During a ureteroscopic lithotripsy procedure, a ureteroscope may enter the kidney 70 through the ureter and use stone-retrieval basket(s) and/or lithotripter(s) to relocate and break down kidney stones, respectively. For example, a lithotripter can be deployed through a working channel of the ureteroscope and used to break the stone 1 into fragments, which may be aspirated through the catheter 48, catheter handle 9, and outflow port 81 to a collection reservoir.

Irrigation and/or aspiration can be managed to produce desirable flow characteristics resulting in desirable distension conditions for the target organ/anatomy. In some embodiments, irrigation (inflow) enters the treatment site through a first medical device (e.g., percutaneous-access instrument 40 and/or catheter 48 disposed therein). In some embodiments, the percutaneous-access instrument 40 and/or catheter 48 can be inserted into the treatment site antegrade of an object (e.g., kidney stone) 80 to be removed, whereas another medical instrument (e.g., endoscope) can be inserted into the treatment site retrograde of the object 1. Although Figure 8B shows the irrigation fluid 3 as provided to the treatment site percutaneously, in some implementations, irrigation may be provided through a medical instrument that accesses the treatment site through direct entry (e.g., via the urinary tract). The aspiration catheter 48 may be robotically controlled as described above with reference to Figures 1, 2, and 4. Accordingly, aspects of the methods, devices, and systems described below can be employed robotically in some embodiments.

In some embodiments, the irrigation and/or aspiration rate(s) can be modulated to improve stone displacement or stabilization or to intentionally create turbulence so that the irrigation reaches all corners of the treatment site. For example, a gentle alternating cycle of irrigation and aspiration can create a lavage-like effect to preferentially pull large stone debris away from calyces and towards the aspiration site(s). Alternatively, short pulsatile inflow and outflow could be used to create turbulence and ensure that smaller and lighter stone fragments do not settle on the floor of the treatment site, but instead remain floating in the irrigation fluid and eventually are aspirated with the outflow.

The percutaneous-access catheter 48 can be an articulable catheter that is introduced via percutaneous access into the treatment site (e.g., the calyx network of the kidney 70). The catheter 48 can be configured to be able to navigate within the kidney 70. For example, the catheter 48 may be configured to be inserted and retracted into the treatment site and/or to articulate (e.g., bend) therein. In some embodiments, the catheter 48 can include pull-wires for controlling articulation. For example, four pull-wires may be oriented in the four orthogonal directions to enable articulation of the catheter 48. Other methods for permitting articulation of the catheter are also possible. The catheter 48 can include, for example, an aspiration lumen (or channel). The aspiration lumen can be fluidly coupled to a pump/vacuum device (e.g., external pump). The pump/vacuum may generate negative pressure that causes flow from the treatment site into the catheter. The aspiration function may be able to be toggled (e.g., on and off) and adjusted by the user or system. In some embodiments, the aspiration lumen may be used for irrigation as well.

The catheter 48 can provide various functions during an object removal procedure, such as stone stabilization during lithotripsy. For example, if the stone 1 is larger than the aspiration lumen of the catheter, the stone can be held at the distal face of the aspiration lumen, thus stabilizing the stone while it is broken down to dust and smaller fragments. Active aspiration may hold the stone to the distal face of the catheter 48. In some cases, a stone being extracted can substantially seal off the catheter 48, thereby causing the stone to be held by the catheter due to the pressure differential. This may provide the user with a less-mobile target for lithotripsy. Moreover, the catheter 48 can improve visibility of the treatment site by removing stone dust from the kidney. This can provide the user with improved visibility (e.g., continuously adequate visibility), for example, from an imaging device inserted into the treatment site.

The catheter 48 can remove stone dust and fragments, wherein the fluid flow carries fluid and debris into the catheter 48 for removal therethrough. Generally, the debris may be cleared as it is generated (i.e., while the stone is being broken up). The removal of debris via the catheter 48 can take the place of the removal of fragments via ureteroscopic basketing, which can be relatively time consuming due to the difficulty of closing the basket around the stone, and due to the need to remove and re-insert the ureteroscope during each fragment removal. Therefore, using the catheter 48 for stone removal can result in a more efficient removal procedure. Removing stone debris via the catheter can also reduce the risk of the stone fragment(s) injuring tissue compared to certain alternative stone removal methods, such as removal of stones through the ureter.

The catheter 48 can be used in several ways during a procedure. For example, the catheter 48 can be mobile throughout the procedure. The catheter can navigate around the treatment site to target specific stones/fragments in order to constrain them during lithotripsy, while also aspirating dust/debris. As another example, the catheter 48 can be initially stationary during the procedure and a scope or other device can be used to relocate stones to the catheter 48. The stones may be broken down at the catheter 48. At a later time during the procedure, the catheter 48 may be navigated through the treatment site to pick up remaining debris. As another example, the catheter 48 may be inserted (e.g., percutaneously) only when required.

The stabilizer 190 can serve to hold the percutaneous-access device 40 and maintain alignment thereof in the proper position once the desired position has been achieved. The stabilizer 190 may be secured to the surgical table or other structure in the operating environment. In some cases, the stabilizer 190 may be free-standing. The stabilizer 190 may advantageously be movable so that it can be brought into the proper position where the percutaneous-access device 40 has been placed. To such end, the stabilizer 190 may comprise one or more joints 191 to provide the necessary degrees of movement.

Generally, the catheter 48 and/or associated handle 9 may be configured to travel axially along the axis 801 of the shaft 42 of the percutaneous-access device 40, which establishes the patient tract. Therefore, proper alignment of the catheter 48 with the axis 801 of one of the percutaneous-access device 40 can be critical to facilitate such travel/advancement of the catheter 48. Misalignment between the catheter 48 and the shaft 42 can lead to device damage with respect to the catheter 48 and/or the percutaneous-access device 40 and may further have a negative impact on system fluidics. For example, misalignment of the catheter 48 may cause kinking or other fluid blockage in the catheter and/or in one or more portions of the percutaneous-access device 40. Embodiments of the present disclosure provide solutions for aligning catheters and/or other instruments with percutaneous-access devices such as those similar to the illustrated devices in Figures 8A and 8B. Such alignment solutions can advantageously direct the movement/advancement of an instrument manipulator assembly and/or component(s) thereof (e.g., the catheter handle 9 in Figure 8A) into an aligned orientation with the percutaneous-access device 40.

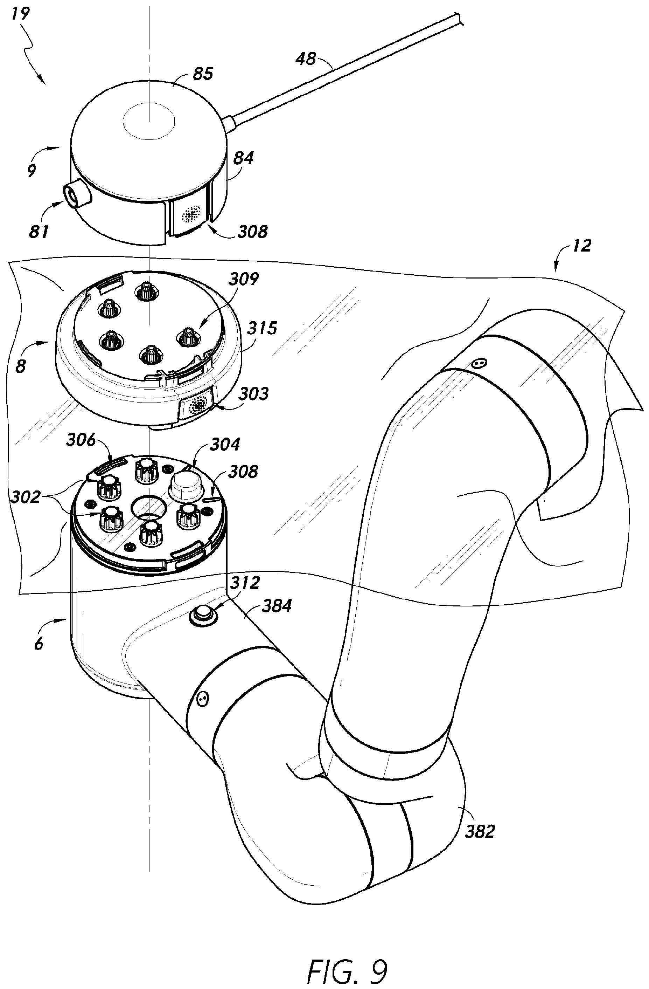

Figure 9 shows an exploded view of an instrument device manipulator assembly 19 associated with a robotic arm 12 in accordance with one or more embodiments. The instrument manipulator assembly 19 includes an instrument driver (e.g., end effector) 6 associated with a distal end of the robotic arm 12. The instrument manipulator assembly 19 further includes an instrument handle 9 associated with a medical instrument 48. The instrument handle 9 can incorporate electro-mechanical means for actuating the instrument 48. For convenience, the instrument 48 is described herein as an aspiration catheter, but the instrument 48 may be any type of medical/surgical instrument. Description herein of upward-facing and downward-facing surfaces, plates, faces, components, and/or other features or structures may be understood with reference to the particular orientation of the device manipulator assembly 19 shown in Figure 9. That is, although the instrument driver 6 may generally be configurable to face and/or be oriented in a range of directions and orientations, for convenience, description of such components herein may be in the context of the generally vertical facing orientation of the instrument driver 6 shown in Figure 9.

In some embodiments, the instrument device manipulator assembly 19 further includes an adapter component 8 configured to provide a driver interface between the instrument driver 6 and the instrument handle 9. The adapter 8 and/or the instrument handle 9 may be removable or detachable from the robotic arm 12 and may be devoid of any electro-mechanical components, such as motors, in some embodiments. This dichotomy may be driven by the need to sterilize medical instruments used in medical procedures and the inability to adequately sterilize expensive capital equipment due to their intricate mechanical assemblies and sensitive electronics. Accordingly, the instrument handle 9 and/or adapter 8 may be designed to be detached, removed, and interchanged from the instrument driver 6 (and thus the system) for individual sterilization or disposal. In contrast, the instrument driver 6 need not be changed or sterilized in some cases and may be draped for protection.

The adapter 8 can include connectors to transfer pneumatic pressure, electrical power, electrical signals, and/or optical signals from the robotic arm 12 and/or instrument driver 6 to the instrument handle 9. The instrument handle 9 may be configured to manipulate the medical instrument 48 using one or more direct drives, harmonic drives, geared drives, belts and pulleys, magnetic drives, and/or other manipulator means or mechanisms. The robotic arm 12 can advance/insert or retract the coupled medical instrument 9 into or out of the treatment site. In some embodiments, the instrument handle 9 can be removed and replaced with a different type of instrument handle. For example, a first type of handle can be configured to manipulate a catheter, a second type of handle can manipulate an endoscope, a third type of handle can hold an EM field generator, and so on.

The end effector (e.g., instrument driver) 6 of the robotic arm 12 can include various components/elements configured to connect to and/or align with components of the adapter 8, handle 9, and/or instrument 48. For example, the end effector 6 can include drive outputs 302 (e.g., drive splines, gears, or rotatable disks with engagement features) to control/articulate a medical instrument, a reader 304 to read data from a medical instrument (e.g., radio-frequency identification (RFID) reader to read a serial number from a medical instrument), one or more fasteners 306 to attach the medical instrument 9 and/or adapter 8 to the instrument driver 6, markers 308 to align with an instrument that is manually attached to a patient (e.g., an access sheath) and/or to define a front surface of the device manipulator assembly. In some embodiments, a portion (e.g., plate) 315 of the adapter 8 can be configured to rotate/spin independently of one or more other components of the adapter 8 and/or driver 6 when coupled to the driver 6.

In some configurations, a sterile drape, such as a plastic sheet or the like, may be disposed between the instrument driver 6 and the adapter 8 to provide a sterile barrier between the robot arm 12 and the instrument 48. For example, the drape may be coupled to the adapter 8 in such a way as to allow for translation of mechanical torque from the driver 6 to the adapter 8. The adapter 8 may generally be configured to maintain a seal around the actuating components thereof, such that the adapter 8 provides a sterile barrier itself. The use of a drape coupled to the adapter 8 and/or more other component(s) of the device manipulator assembly 19 may provide a sterile barrier between the robotic arm 12 and the surgical field, thereby allowing for the use of the robotic cart associated with the arm 12 in the sterile surgical field. The driver 6 may be configured to be coupled to various types of sterile adapters that may be loaded onto and/or removed from the driver 6 of the robotic arm 12. With the arm 12 draped in plastic, the physician and/or other technician(s) may interact with the arm 12 and/or other components of the robotic cart (e.g., screen) during a procedure. Draping may further protect against equipment biohazard contamination and/or minimize clean-up after procedure.

Although the particular adapter 8 shown in Figure 9 may be configured for coupling with a catheter handle 9, such as an aspiration catheter handle, adapters for use with device manipulator assemblies in accordance with aspects of the present disclosure may be configured for coupling with any type of surgical or medical device or instrument, such as an endoscope (e.g., ureteroscope), basketing device, laser fiber driver, or the like.

### Alignment Devices and Assemblies

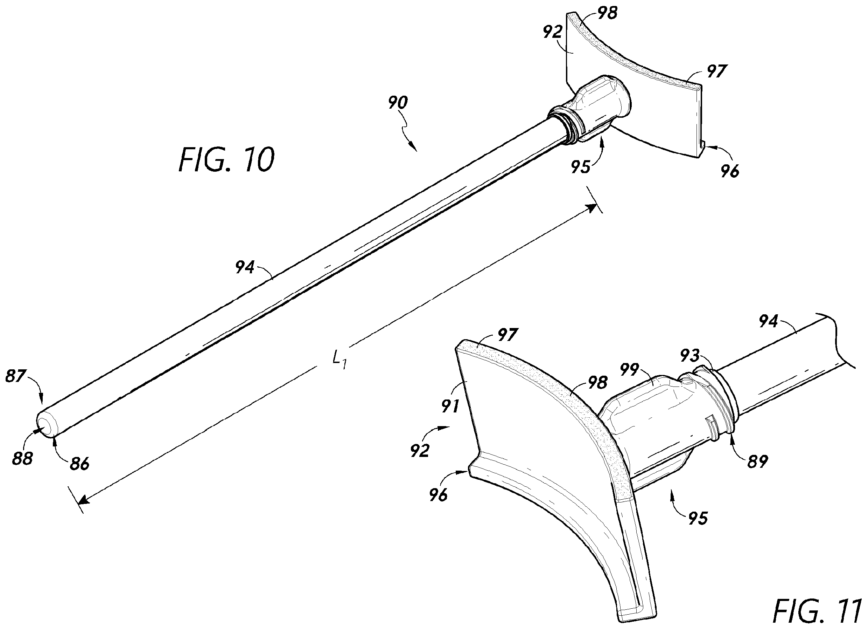

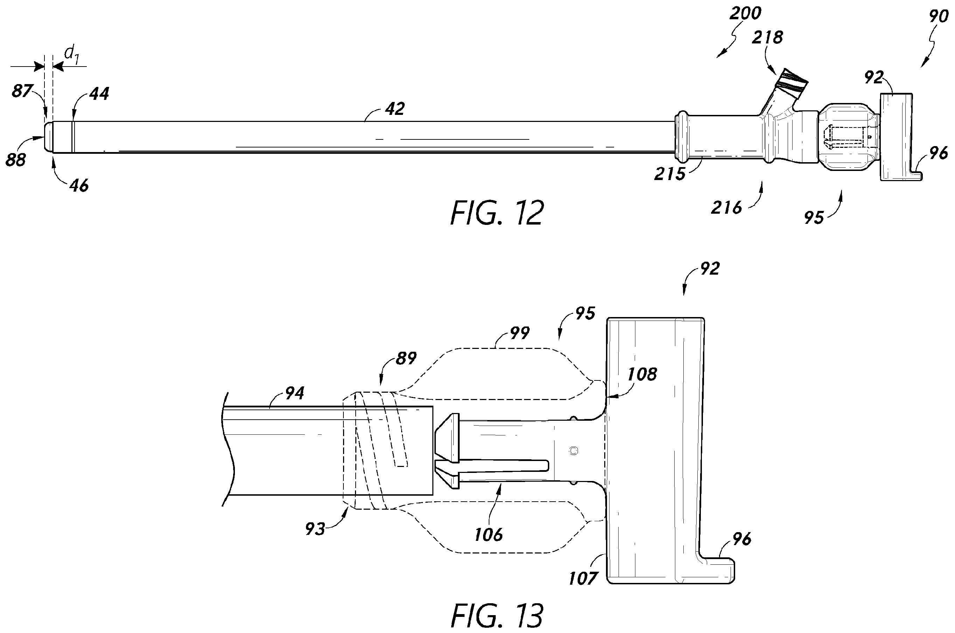

Figure 10 shows a distal and side isometric view of an alignment device 90 in accordance with one or more embodiments of the present disclosure and in accordance with the present claimed invention. Figure 11 shows a proximal and side isometric view of a proximal portion of the alignment device of Figure 10 in accordance with one or more embodiments. Figure 12 shows a side view of the alignment device 90 of Figures 10 and 11 disposed in a percutaneous-access device 200 (e.g., outer sheath component of a percutaneous-access device/assembly) in accordance with one or more embodiments. Figure 13 shows a cut-away view of a proximal portion of the alignment device 90 in accordance with one or more embodiments. The alignment device 90 is configured to facilitate registration of a sterile adapter or other component of an instrument manipulator assembly to a desired alignment position and/or path through feature-matching component alignment. The alignment device 90 includes a blunt tip 87 to reduce the risk of tissue damage during alignment, as well as threaded or other features configured to facilitate secure installation of the alignment device 90 in a percutaneous-access device and/or an outer sheath component thereof.

During an alignment process, an inner sheath component (e.g., inner sheath component 220 shown in Figures 7A and 7B) of the percutaneous-access device may not be present and/or coupled with the outer sheath component 200, such that the outer sheath component 200 may instead receive the alignment device 90 at least partially therein for implementation of alignment procedures as described in detail herein. The inner sheath component 220 may be coupled to the outer sheath component 200 subsequent to the alignment procedure/process, after the proper alignment rail/path and/or position of the robotic instrument manipulator assembly has been determined.

As shown in Figure 12, the alignment device 90 is configured to be coupled to and/or disposed in some manner at least partially within at least a portion of a percutaneous-access device 200. For example, the alignment device 90 may include an elongate rod 94 (see Figure 10) configured to fit within an outer shaft of a percutaneous-access device 200, such that a distal end/tip 87 thereof protrudes some distance from a distal end 46 of the percutaneous-access device/shaft 42.

The elongate rod 94 may serve to provide an atraumatic contact tip 87 for a percutaneous-access device when the percutaneous-access device is disposed in the patient in connection with a medical procedure, such as a kidney stone removal procedure or the like. The atraumatic/blunt tip 87 may include an at least partially flat axial face 88 and/or rounded corner/edge portion(s) 86 to provide the blunt form that is less potentially damaging to tissue at the treatment site than the distal end of the percutaneous-access device 200 alone. The length *L₁* of the elongate rod 94 can serve to place the distal tip 87 thereof distal to the distal opening 46 of the percutaneous-access device 200 and/or outer shaft 42 thereof. In some embodiments, the elongate rod 94 has a length of approximately 7.1-7.2 inches (1 inch ≈ 2.54 cm). The length *L1* may advantageously be determined relative to a length of the tube portion of a percutaneous-access device in which the elongate rod 94 is configured to be disposed. For example, the length *L₁* may advantageously be longer than such tube by an amount between 0-5mm. For example, the length *L₁* may be longer than the tube of the percutaneous-access device by a distance between 3-5mm.

It may be desirable to limit the protrusion distance/length *d₁* of the elongate rod 94 from the shaft 42 to prevent undesirable contact with and/or movement of the patient anatomy by the distal end 87 of the rod 94. For example, in some embodiments, the protrusion distance *d₁* of the rod 94 past the distal end 204 of the outer sheath component 200 may be approximately 5 mm or less. With such a short protrusion distance *d₁,* the edges of the distal end 46 of the shaft 42 can substantially blend and/or smoothly transition with the tip 87 (e.g., side(s) 86) of the rod 94. That is, the distal end 46 of the shaft 42 may have at least partially rounded or tapered edges that may transition against the rounded or tapered sides 86 of the distal tip 87 of the rod 94. The presence of the blunt tip 87 distal to the distal end 46 of the shaft 42 may reduce impact/damage from direct axial contact with the profile of the tubular shaft 42, wherein the flat contact surface 88 is less traumatic potentially to biological tissue than an alternative ring-like interface of the shaft 42 that may be present in the absence of the rod 94 of the alignment device 90.

The alignment device 90 may further be configured to provide a proximal fluid seal with the outer sheath component 200, which may be achieved using a proximal hub portion 95 (also referred to herein as a hub structure, hub component, and/or hub member), which may be similar in some respects to similar hub components of certain inner sheath components of percutaneous-access devices in accordance with aspects of the present disclosure.

The alignment device 90 further includes an alignment structure/member 92, which may comprise a certain structure or form having a shape and/or configuration designed to engage or interface with a component of a robotic instrument manipulator assembly to facilitate/guide engagement and alignment of one or more components of the instrument manipulator assembly with the alignment structure/member 92 to thereby determine an alignment axis or path associated with the percutaneous-access device in which the alignment device 90 is disposed and/or determine an alignment position of the instrument manipulator assembly and/or movement axis or path therefore. The alignment structure/member 92 can have a concave, dish-type shape/form in accordance with the present claimed invention.

As referenced above, the rod 94 has a blunt tip 87 that can reduce or remove the risk of tissue damage caused by sudden sharp movements of the distal end of the percutaneous access device in which the rod is configured to be disposed when it contacts tissue. The atraumatic shape/form of the distal tip 87 of the rod 94 can prevent trauma to biological tissue from dislodging and/or manipulation of the percutaneous access device during an alignment process. For example, the percutaneous-access device, or the outer sheath component thereof, may comprise relatively thin walls. For example, such walls may have a thickness of approximately 10/1000 of an inch or other similarly thin dimension. Generally, when attempting to align the robotic instrument manipulator assembly with the percutaneous-access device, the robotic arm (e.g., the instrument manipulator assembly or other portion thereof) may be bumped against the access device, which can cause sharp movements and/or impacts of the distal end and/or other portion(s) of the percutaneous-access device against biological tissue of the patient. Therefore, the rod 94 and/or distal end 87 thereof can mitigate and/or reduce any cutting or damaging of tissue due to the thin wall thickness of the access device/sheath. That is, by having the blunt end 87 stick-out/project from the access device/sheath a relatively minimal amount, as shown in Figure 12, if the distal end 46 of the percutaneous-access device 200 moves during the alignment process, the contact with any biological tissue will primarily be with the tip 87 of the elongate rod 94 rather than the distal end 46 of the sheath 42, at least initially, which may prevent and/or reduce the potential damage to the biological tissue.

The alignment device 90 is configured to be disposed in an outer sheath component (e.g., the outer sheath component 200) of a percutaneous-access device, wherein the alignment device 90 may include a sealing hub 95 that is configured to match the sealing method of an inner sheath component of the percutaneous-access device, such that insertion of the sealing hub 95 and engagement with the proximal hub portion 216 of the percutaneous-access device 200 can be implemented to form a proximal fluid seal between the hub 95 of the alignment device 90 and the proximal hub 216 of the percutaneous-access device. For example, the hub 95 may have certain tapered contact features 93 configured to create a seal via a taper-to-taper interaction with corresponding surfaces/features of the inside of the hub 216 of the percutaneous-access device. Although shown as tapered features 93, in some embodiments, the sealing surfaces/features may be axially-flat/blunt surfaces that provide a sealing seating against which corresponding flat/blunt surfaces of the hub 216 can be pressed/placed to provide a fluid seal.

The alignment structure 92 can be shaped or formed to facilitate and/or guide alignment by a user of a sterile adapter surface or other component of an instrument manipulator assembly into desirable contact with the alignment structure 92. For example, the shape of the alignment structure 92 and/or engagement lip 96 associated therewith can promote or ensure that correct alignment of the robotic instrument manipulator assembly occurs when the manipulator assembly (e.g., circumferential surface thereof) is docked with the alignment structure 92 of the alignment device 90 and rotated to align certain alignment markings or structures of the manipulator assembly (e.g., of an adapter component thereof) with visual and/or tactile markings 97 associated with the alignment structure 92. For example, such alignment marking(s) may be visible, disposed, printed, and/or otherwise present on an upper surface 98 of the alignment structure 92. In some embodiments, the upper surface 98 may be substantially flat over a length thereof. The lip 96 may be inwardly-projecting and circumferential with respect to the curvature of the proximal surface 91 of the alignment structure 92, as shown.

With the elongate rod 94 disposed within the outer shaft 42, some amount of clearance between the outer surface of the rod 94 and the interior surface of the outer shaft 42 may be present through which irrigation fluid may be provided to the treatment site via the port 218 of the outer sheath component 200. However, it may be desirable to prevent fluid from leaking out the proximal end of the hub 216, such as in a case where fluid is being irrigated to the treatment site (e.g., kidney) or fluid conditions of the treatment site are to be maintained with little or no passive outflow. For example, with respect to kidney procedures, outflow of fluid from the kidney through the proximal end of the hub 216 may cause the kidney to at least partially collapse due to fluid loss and/or desired distention of the kidney may be lost or reduced. Therefore, the sealing contact surface(s) 93 of the hub 95 can serve to prevent such undesirable fluid leakage. The hub 95 may further comprise certain threading features 89 and/or manual turning features/fins 99 to assist in coupling the sealing hub 95 to the hub 216 of the percutaneous-access device. For example, the threads 89 may engage with corresponding threads inside the hub component 216 of the percutaneous access device. The user may manually handle and rotate the hub component 95 in order to couple and seal the alignment device 90 to the outer sheath component 200. In some embodiments, the distal end portion 46 of the outer shaft 42 includes a marker band or other feature 44 (e.g., laser marker band), which may be used to detect when the shaft 42 is a certain distance into the target chamber, vessel, or other treatment site. For example, the marker band 44 may become visible on an endoscope image, thereby indicating the distance that the shaft 42 has advanced into the treatment site (e.g., target calyx of the kidney).

The alignment structure 92 may have a dish-type form that is proximally concave to conform to the convex curvature of the surface of a component of an instrument manipulator assembly. For example, the concave form 92 may be dimensioned/shaped to interface against an outward radiused/convex surface of an adapter component of an instrument manipulator assembly as described herein.

In some embodiments, the alignment structure 92 further includes a lip feature 96 that projects radially inward with respect to an axis of curvature of the alignment structures 92. Although the lip feature 96 is shown running along a bottom portion of the alignment structure 92, it should be understood that lip features in accordance with aspects of the present disclosure may be disposed and/or positioned at or along a bottom portion or side, top portion or side, and/or any other portion or side of the alignment structure 92. The lip feature 96 may be configured to provide a seating surface against which an underside of a component of the instrument manipulator assembly may be placed or rested to provide vertical alignment with the alignment device 90.

The feature 92 may have a dish or sheet-type form as shown, or may have any other shape or configuration designed to mechanically/physically engage or interface with a component of an instrument manipulator assembly of a robotic system. With respect to the illustrated configuration, the concavity of the alignment structures 92 provides for mechanical engagement with one or more components of the instrument manipulator assembly (e.g., sterile adapter component). However, it should be understood that some embodiments may incorporate other types of engagement features, such as one or more magnets, lasers or other optical devices/sensors, and/or other types of features that can promote and/or direct alignment of the proximal portion of the alignment device 90 with a component of an instrument manipulator assembly.

The top surface 98 of the alignment structure 92 can be printed or otherwise marked to aid with aligning with the top surface of the corresponding component of the instrument manipulator assembly of the robotic system (e.g., top surface of a rotating plate portion of an adapter component of the instrument manipulator assembly). The hub 95 may include a partial taper feature 93 that is configured to hold a fluid seal when attached to the outer sheath component 200.

In some embodiments, the alignment structure 92 may be a separate component from the hub 95 and/or rod 94 of the alignment device/assembly 90. For example, the alignment structure 92 may be removable from of the hub 95. In some implementations, the elongate rod 94 may be coupled to the hub component 95 and inserted into the outer sheath component 200 together as a combined assembly, wherein the alignment structure 92 may be attached to the hub 95 after placement in the outer sheath component 200 in anticipation of alignment. In some embodiments, the rod 94 may be cured to the hub 95 in some manner, such as through ultraviolet radiation or the like.

The alignment structure 92 may comprise a male connector 106 that may project axially distally with respect to the rod and hub and be configured to be received by a corresponding female cavity of the hub 95. The projection 106 can allow for the alignment structure 92 to be decoupled from the remainder of the alignment device (e.g., the assembly of the hub 95 and rod 94). The projection 106 may be a snap-fit type connector/projection and may have a length such that it can lock into place without contacting the proximal end of the rod 94 when received in the hub 95 (e.g., in a female receptacle thereof), as shown in Figure 13. For example, the distal side 107 of the alignment structure 92 may bottom-out on the proximal side 108 of the hub 95 prior to contact of the connector 106 with the rod 94. With the separably-coupled alignment structure 92 and hub 95 connected as shown in Figure 13, in some embodiments, the alignment structure 92 may have some degree of freedom to rotate axially within the hub 95. However, it may be desirable to limit the ability of the alignment structures 92 to rotate to avoid undesirable rotation when approximating and mating the instrument device manipulator assembly to the alignment structure 92.

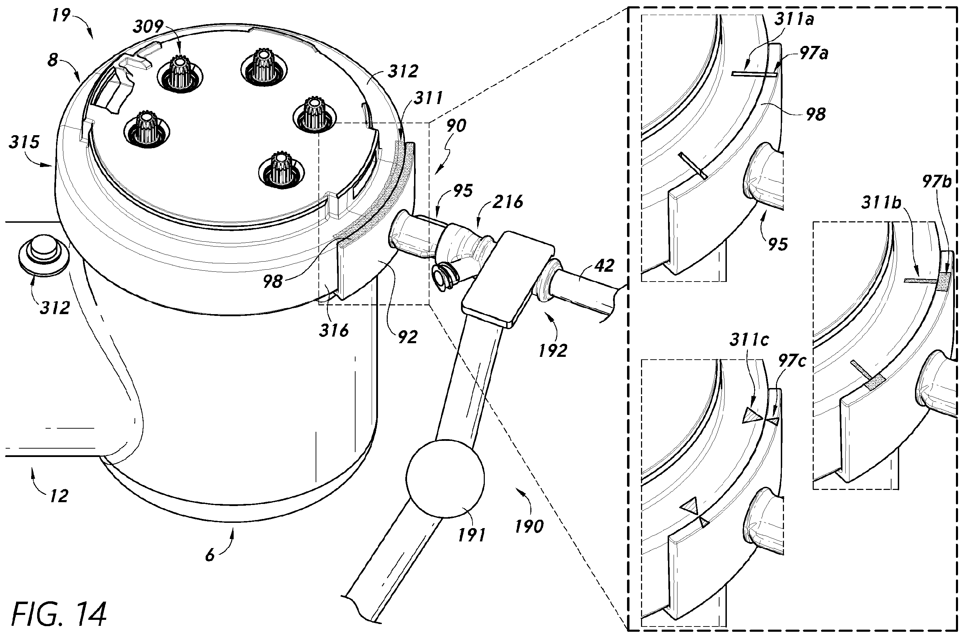

Figure 14 shows an alignment device 90 having an alignment structure 92 engaged with one or more surfaces and/or features of an instrument manipulator assembly 19 in accordance with one or more embodiments of the present disclosure. In the image of Figure 14, the alignment structure 92 is engaged/interfaced with a rotating plate component 315 of the instrument device manipulator assembly 19 shown, which may include an instrument driver 6 and adapter 8 at the stage in the process associated with alignment of the manipulator assembly 19 with the alignment device 90. At this stage, the instrument handle/base (e.g., catheter handle) may not be coupled to the assembly 19. As with other embodiments of the present disclosure, although an adapter component 8 is shown coupled to the instrument driver 6, it should be understood that engagement/interfacing of the alignment device 90 with the instrument manipulator assembly 19 may be with respect to any component of the assembly.

Once the rotating plate 315 or other component of the manipulator assembly 19 is brought into mating contact with the alignment structure 92, such as by positioning an outer surface 316 of the rotating plate 315 against the concave proximal surface 91 of the alignment structure 92, such that an under surface of the rotating plate 315 is seated on the vertical-alignment lip 96, the rotating plate 315 may be rotated axially to align one or more alignment markers/features 311 associated with the manipulator assembly 19 (e.g., on an upper surface 312 of the rotating plate 315) into axial alignment as indicated by one or more markers or indicators 97 associated with the alignment structure 92. For example, such feature(s) 97 may be on or associated with the upper surface 98 of the alignment structure 92. Although Figure 14 and other figures and/or embodiments illustrated and/or described herein relate to a bottom lip feature 96 configured to have an underside of a component of the instrument device manipulator assembly 19 seated/placed thereon, it should be understood that such lip feature(s) may be implemented as upper-lip features, wherein such feature(s) may be associated with the top side/portion of the alignment structure 92 and configured to allow the alignment structure 92 to be placed or rested on an upper surface of a component of the manipulator assembly 19 (e.g., upper surface 312 of the rotating plate 315). That is, in such embodiments, the instrument manipulator assembly 19 may be brought up against one or more upper lip features of the alignment structure 92.

Rotation of the rotating plate 315 may be substantially independent of rotation of portions of the instrument driver 6. For example, axially rotating the rotating plate 315 of the adapter 8 may not result in commensurate rotation of an outer housing of the driver 6. Rotation of the rotating plate 315 may, however, result in rotation of certain other features of the adapter 8 and/or assembly 19, such as the drivers/gears 309, and/or other components, as well as certain corresponding interface components of the driver 6.

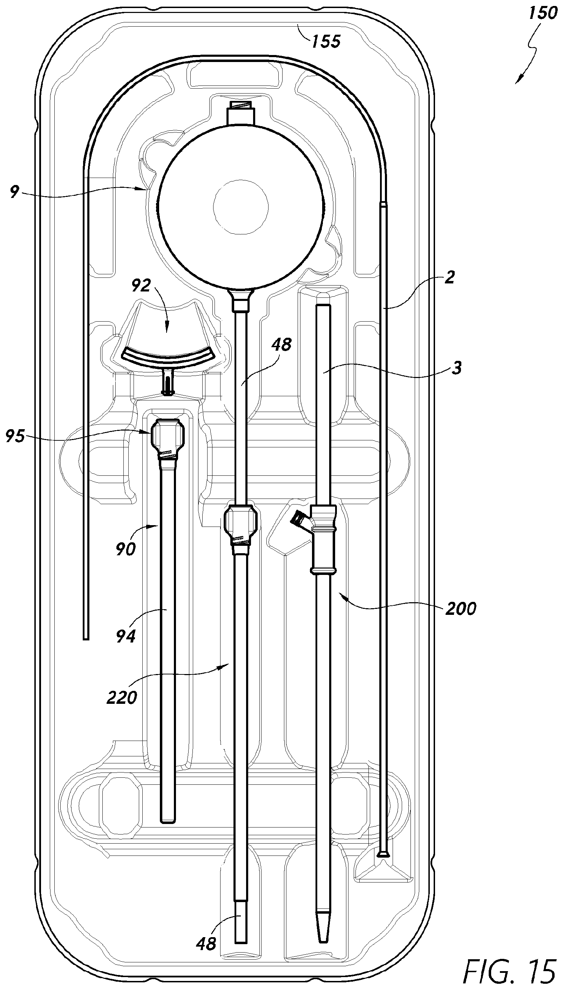

Figure 15 shows a kit 150 for a percutaneous-access system in accordance with one or more embodiments. The kit 150 may advantageously be sterilized for surgical use. In some embodiments, shrink wrap or other sanitary covering may envelop the kit 150 prior to use thereof. Such covering may be air- and/or fluid-tight in some embodiments.

The kit 150 may comprise a component-retention form or structure 155, referred to as a "tray" herein, such term being used according to its broad and ordinary meaning. The tray 155 may be implemented as a single unitary form, and/or may be composed of a plurality of pieces or components integrated together to form the retention tray 155. For example, the tray 155 may include one or more recesses, wells, channels, cavities, openings, depressions, indentations, and/or the like configured and/or shaped to receive and/or have placed therein one or more portions of the various components retained in the retention form/structure 155. Furthermore, the tray 155 may include certain pressure-, tension-, and/or friction-type fittings/features configured to retain the various components and reduce the tendency of such components/portions to become disengaged or dislodged from the tray 155. That is, in some embodiments, a certain amount of force may need to be exercised to withdraw one or more of the components or portions thereof from the tray 155, such that such components/portions are at least partially secured in their respective positions/wells in the kit 150.

The kit 150 may include one or more of a catheter 48, catheter handle 9, outer sheath component 200, inner sheath component 220, dilator 3, alignment device 90, and/or dilator and sheath assembly 2, as illustrated. However, it should be understood that the kit 150 may include additional components not illustrated in Figure 15 and/or one or more of the components shown in Figure 15 may be omitted in some embodiments. As shown in Figure 15, in some embodiments, the catheter 48 may be stored/retained in the retention tray 155 at least partially within a lumen of the inner sheath component 220. It should be understood that the inner sheath component 220 and the outer sheath component 200 may be components of a percutaneous-access device assembly, as described in detail herein. Furthermore, in some embodiments, the dilator 3 may be secured in the retention tray 155 at least partially within a lumen of the outer sheath component 200, as shown. **In** some embodiments, the alignment device 90 may be configured in the kit 150 in a detached configuration, wherein the alignment structure 92 is disposed separately from the assembled elongate rod 94 and hub 95 components, which may be coupled together or separate in the kit 150. For example, the rod 94 may be joined with the hub 95 with a cured adhesive, such as an ultraviolet-radiation-cured adhesive. In some embodiments, the alignment device 90 is disposed in the retention structure/form 155 in an assembled configuration, wherein the alignment structure 92 is mechanically coupled to one or more of the hub component 95 and the elongate rod component 94. In embodiments in which the alignment structure 92 and the remainder of the alignment device 90 are disposed in the tray 155 separately as shown in Figure 15, such components may be held in a common cavity or portion of the tray 155.

### Alignment Processes

Figures 16-1, 16-2, 16-3, 16-4, and 16-5 show a flow diagram illustrating a process 1600 for aligning a medical instrument and/or instrument manipulator assembly in accordance with one or more embodiments. Figures 17-1, 17-2, 17-3, 17-4, and 17-5 show certain images corresponding to various blocks, states, and/or operations associated with the process 1600 in accordance with one or more embodiments of the present disclosure.

Prior to execution of the process 1600, certain preliminary processes and/or operations may be performed. For example, prior to the operations associated with block 1602, the process 1600 may involve accessing a percutaneous-access kit, which may be similar in certain respects the kit 150 shown in Figure 15, described above. Preliminary processes/operations may further involve percutaneously accessing the target treatment site, such as the internal anatomy of a kidney of the patient, and dilating the access path thereto. For example, a needle may first be used to puncture the skin and tissue leading to the treatment site (e.g., access through a target papilla into a target calyx of a kidney), after which a guidewire may be passed through and/or along the needle. A dilator can be passed over the guidewire to dilate the percutaneous path to the treatment site. After access and dilation, an outer sheath component 200 of a percutaneous-access device assembly may be inserted such that the distal end 46 thereof is disposed at least partially within the treatment site. After placement of the outer sheath component 200, such device/component may be secured in its desired placement using a stabilizer structure 190.

At block 1602, the process 1600 involves inserting an alignment device into the percutaneous-access device/sheath. For example, the percutaneous-access device may comprise an outer sheath component 200 of a percutaneous-access device assembly according to certain embodiments of the present disclosure. Insertion of the alignment device 90 may involve advancing an elongate rod portion 94 of the alignment device 90 through a lumen of a shaft 42 of the outer sheath component 200. The alignment device 90 may be inserted in an assembled form in which the elongate rod 94, hub 95, and alignment structure 92 are coupled together, or may be inserted in a configuration in which the elongate rod and hub 95 are coupled together and inserted as an integrated assembly, whereas the alignment structure 92 is clipped or otherwise secured to the hub 95 after placement and securing of the hub 95 and rod 94 assembly in/to the outer sheath component 200 and/or hub 216 thereof. The alignment device 90 and/or hub component 95 thereof can be mechanically secured/coupled to the outer sheath component 200, such as by inserting the rod 94 at least partially into the component 200 and screwing the hub 95 into the outer sheath hub 216 to tighten and seal the hub 95 against one or more internal surfaces of the outer sheath hub 216. In some implementations, once the hub 95 has been securely coupled to the sheath hub 216, the alignment structure 92 may be rotated so that it is approximately parallel ground with respect to top and/or bottom surfaces thereof. **In** some implementations, no such rotation step is implemented. For example, such rotation may not be necessary as the instrument device manipulator assembly 19 may force such rotational alignment of the alignment structure 92 to match the plane of the instrument manipulator assembly 19 and/or one or more components thereof to manually force the alignment structure 92 into alignment with the manipulator assembly 19.

At block 1604, the process 1600 involves coupling or otherwise mating or engaging one or more components of a robotic instrument device manipulator assembly 19 with the alignment structure 92 of the alignment device 90. Such operation(s) may involve manually positioning the robot arm 12 such that the manipulator assembly 19 interfaces, mates, or otherwise engages with the alignment structure 92. For example, a projecting lip feature 96 of the alignment structures 92 can engage with a corresponding bottom surface of a component of the instrument manipulator assembly 19, such as an adapter plate component thereof. For example, a rotatable top plate component of the adapter 8 may physically interface with the alignment member 92 in the ways described.

According to some implementations, operation(s) associated with block 1604 can involve placing the robotic system 10 in an arm-alignment pose/configuration. For example, a clutch-type mechanism may be engaged to allow for manual repositioning of one or more portions of the arm 12 in a relatively free manner. In some embodiments, such an alignment mode of the robotic system 10 may be engaged/initiated substantially automatically and/or in response to one or more triggering events associated with the relevant procedure(s)/process(s). In some embodiments, a manual manipulation mode of the arm 12 may be initiated using one or more inputs on the arm (e.g., buttons, touch sensors, etc.) and/or applying a force to the arm that exceeds a threshold.

With respect to the image 1703 associated with the mating of the instrument device manipulator assembly 19 with the alignment structure 92, such mating may involve manually articulating, moving, and/or arranging the robot arm 12 in any suitable or desirable way. Such arrangement/configuration of the various parts of the robot arm 12 can indicate and/or be based on certain kinematic information/data associated with the robot arm. For example, kinematic information/data on which positional and/or alignment configuration of the robot arm 12 may be based can include actuator position data, actuator velocity data, actuator sequencing information, and/or other data associated with the particular configuration of the robot arm 12 when engaging with the alignment structure/member 92.

In certain embodiments, the robot arm 12 can have seven joints 24, and thus, provide seven degrees of freedom. However, any number of joints can be implemented with any number of degrees of freedom. Generally, multiple arm joints can result in a multitude of degrees of freedom, allowing for "redundant" degrees of freedom. Redundant degrees of freedom allow the robotic arm 12 to position the instrument driver 6 at a specific position, orientation, and/or trajectory in space using different linkage positions and/or joint angles (certain example arm angles are shown in the image 1703). In some embodiments, the instrument driver 6 can be configured to engage with and/or control a medical instrument, a device, an object, and so on. The freedom of movement of the arms 12 can allow the robotic system 10 to position and/or direct a medical instrument (e.g., catheter) from a desired point in space and/or allow a physician to move the arms 12 into a mating configuration with the alignment device 90 and thereby into alignment with the axis of the shaft 42 of the percutaneous-access device 200.

The various arm segments 23 and/or associated actuators can be moved/adjusted to achieve the mating position shown in image 1703, wherein the manipulator assembly 19 is aligned with the sheath angle *θ₅* of the percutaneous-access device 200. For example, due to user variability with respect to the alignment of the shaft 42, the sheath angle *θ₅* can be variable across procedures. In order to align with the sheath angle *θ₅*, the distal arm segment 23d can be moved to a corresponding angle *θ₄.* The height offset *H₁* of the arm support 17 within the track 20 can further be adjusted to achieve the desired position of the manipulator assembly 19.

At block 1606, the process 1600 involves rotating a rotatable component 315 of the instrument device manipulator assembly 19 to align one or more markings or other visual indicators 311 associated therewith with corresponding alignment marker(s) associated with the alignment structure 92 (e.g., on a top surface thereof as shown in Figure 17-2). In some embodiments, the operation(s) associated with block 1606 involve matching a pad print on a top surface of the alignment tool 92 with corresponding marking(s) of the rotating component 315 of the manipulator assembly 19. The rotating component 315 may comprise a top plate of an adapter component 8 that is coupled to the instrument driver 6 associated with the distal end of the robotic arm 12. Rotating the top plate of the adapter component (or other rotatable component of the adapter 8 and/or manipulator assembly 19) can be implemented to bring the marking(s) 311 into alignment with the marking(s) 97.

At block 608, the process 1600 involves determining a virtual rail and/or alignment path associated with the percutaneous-access device 200 based at least in part on the position, orientation, and/or configuration of the robotic arm and/or instrument device manipulator assembly 19 as interfaced with the alignment device 90. Such rail 170 and/or access path may advantageously account for a vertical offset (with respect to the illustrated orientation in Figure 17-2) between the interface position 171 of the manipulator assembly 19 relative to the position/axis of a catheter 48 that will be (or in some embodiments already is) coupled to the adapter 8 and/or other component(s) of the manipulator assembly 19. The rotating component 315 may be rotated/rotatable clockwise and/or counterclockwise, depending on the embodiment or implementation.

In some implementations, once the robotic arm 12 and instrument manipulator assembly 19 are in the proper alignment, a snapshot may be taken with respect to the position(s) of the arm 12 and/or manipulator 19 to determine and/or record a position thereof in space. Such position determination/measurement may be made using any position sensing/determination scheme, including electromagnetic position sensing, or based on robotic arm position data.

Alignment of the instrument manipulator 19 with the alignment device 90, via the use of the alignment structure 92, may be geometrically defined by the six degrees of freedom of movement for a body within three-dimensional space. That is, if the alignment device 90 is considered to be a fixed point in space, the alignment of the manipulator assembly 19 with the alignment device 90 can be specified by providing values for the six degrees of freedom of movement of the manipulator assembly 19 with respect to the alignment device 90. These degrees of freedom may include the positions (e.g., forward/backward (the X-axis), left/right (the Y-axis), up/down (the Z-axis) as illustrated in image 1703) and/or the orientation (e.g., pitch (rotation around the Y-axis), yaw (rotation around the Z-axis), and roll (rotation around the X-axis)) of the manipulator assembly 19. Although the positional axes (X-axis, Y-axis, and Z-axis) may be defined, located, and/or oriented in various different manners, the present disclosure may generally refer to these axes as illustrated in image 1703 in some contexts herein.

Aspects of the present disclosure relate to methods and techniques for placing the manipulator assembly 19 into an alignment position/orientation with respect to the alignment device 90 to align the manipulator assembly 19 with the alignment device 90. The surgical robotic system 10 may record the spatial position and/or orientation of the manipulator assembly 19 during alignment so that the surgical robotic system 10 is aware of the spatial position and/or orientation of the percutaneous-access device 200 during a surgical procedure.

Alignment of the manipulator assembly 19 with the alignment device 90 may also be defined by the alignment of one or more axes of the manipulator assembly 19 with one or more axes of the percutaneous-access device 200, as indicated by the engagement with the alignment device 90. For example, the percutaneous-access device 200 may define an axis (which may be referred to as a receive axis herein) along which the percutaneous-access device is configured to receive a surgical instrument 48 (e.g., aspiration catheter). In some implementations, the determined rail/path 170 may be or correspond to an axis of the access/outer sheath component 200. Additionally or alternatively, the determined alignment path may indicate and/or guide a nonlinear path of the robotic arm into a desired alignment with the percutaneous-access device 200.

Determination of the alignment path 170 (e.g., "virtual rail") can be based on kinematic information associated with the robotic arm when engaged with the alignment structure/member 92. For example, any of the illustrated angles, aspects, positions, and/or configurations relating to the image 1703 of the robotic cart/system 10 in Figure 17-1 may be associated with certain configuration data that may be used to determine the alignment path.

At block 1610, the process 1600 involves retracting the robotic arm 12 and/or instrument device manipulator assembly 19 away from the sheath component 200 and/or alignment device 90 and alignment structure 92. Such retraction of the robotic arm may allow for reconfiguration of the percutaneous access sheath 200 and may be associated with an end to the alignment stage of the process 1600. That is, the operation(s) associated with block 1610 may be triggered and/or initiated after recording/registration of the alignment position, rail, and/or path 170 as related to block 1608 and in accordance with the alignment process. When or after the robotic arm 12 is retracted from the sheath component 200 and/or alignment device 90, the rotatable component 315 of the instrument device manipulator assembly 19 (e.g., component of the adapter 8) may be rotated by some amount to align one or more components thereof with the sheath component 200. For example, in some implementations, the rotatable component may be rotated by 90° to align with a catheter hub/handle 9 such that a catheter 48 thereof is in alignment with the sheath 200 when the catheter handle 9 is attached to the adapter 8.

At block 1611, the process 1600 involves removing the alignment device 90 from the outer sheath component 200 and inserting the inner sheath component 220 into the outer sheath component 200 to form the percutaneous-access device/assembly 40, wherein a gap between the inner and outer shafts of the inner and outer sheath components, respectively, may be used to provide irrigation fluid to the treatment site. The inner sheath component 220 can be engaged with the hub 216 of the outer sheath component 200 in such a way as to form a proximal fluid seal, which may be in a manner similar to the seal formed between the hub 95 of the alignment device 95 and the sheath hub 216 as described above. It may be desirable to relatively quickly exchange the alignment device 90 with the inner sheath component 220 in order to reduce any proximal fluid leakage from/through the proximal end of the hub 216 in the time during which no inner hub is fluidly sealed therein.

At block 1612, the process 1600 involves inserting an aspiration catheter 48 or other device into the percutaneous-access device 40. Such insertion may be performed manually without the assistance of the robotic system 10 in some implementations. The catheter 48 may be coupled to and/or associated with a handle component 9, which may be configured to be physically coupled to an adapter 8 or other component of instrument device manipulator assembly 19, as described herein. Insertion of the catheter 48 or other instrument into the percutaneous-access device 40 should generally be performed in a careful manner so as to avoid disturbing the access device 40 in a manner that may result in physical harm to the patient and/or obstruction or damage of/to the catheter 48. In some implementations, insertion of the catheter 48 in connection with block 1612 may be performed using the robot arm 12, wherein the handle 9 is coupled to the instrument device manipulator assembly 19 at the time of insertion of the catheter 48. Such insertion and/or advancement may be generally in-line with the determined rail/path 170 at least in part.

At block 1614, the process 1600 involves coupling the catheter handle 9 or other device to the instrument device manipulator assembly 19. For example, advancement of the manipulator assembly 19 and/or associated robotic arm to the coupling position may be via a determined alignment path based at least in part on the registered alignment position/rail.

At block 1615, the process 1600 involves advancing the catheter 48 along the determined virtual rail/path 170 using the robotic arm 12. For example, the robotic system may be configured to move the manipulator assembly 19 along the determined rail/path 170 as determined in connection with block 1608 of the process 1600. The process 1600 may further involve preparing, on a fluidics tower or system, tubing for irrigation and/or aspiration and/or priming such fluid line(s). The process 1600 may further involve transferring an irrigation line to a port 218 of the outer sheath component 200 for providing irrigation to the treatment site. An aspiration line 82 may further be coupled to an aspiration port 81 associated with the handle 9. Such operation(s) may be performed at any stage in the process 1600. The operation(s) associated with block 1615 can involve robotically controlling or constraining movement of the arm 12 along the alignment path, which, as described above, can be determined based on certain kinematic information/data associated with the engagement position of the instrument manipulator assembly 19 with the alignment device 90.

At block 1616, the process 1600 involves aspirating the treatment site within the patient using the catheter 48. For example, fluid may be drawn through the catheter 48 using one or more pumps or vacuum devices, which may pull fluid through a port 81 associated with the catheter handle 9. Such port 81 may be coupled to tubing that is fluidly coupled with one or more components of the fluidics tower/system.

### Additional Embodiments

Depending on the embodiment, certain acts, events, or functions of any of the processes or algorithms described herein can be performed in a different sequence, may be added, merged, or left out altogether. Thus, in certain embodiments, not all described acts or events are necessary for the practice of the processes.

Conditional language used herein, such as, among others, "can," "could," "might," "may," "e.g.," and the like, unless specifically stated otherwise, or otherwise understood within the context as used, is intended in its ordinary sense and is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment. The terms "comprising," "including," "having," and the like are synonymous, are used in their ordinary sense, and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Conjunctive language such as the phrase "at least one of X, Y and Z," unless specifically stated otherwise, is understood with the context as used in general to convey that an item, term, element, etc. may be either X, Y or Z. Thus, such conjunctive language is not generally intended to imply that certain embodiments require at least one of X, at least one of Y and at least one of Z to each be present.

It should be appreciated that in the above description of embodiments, various features are sometimes grouped together in a single embodiment, Figure, or description thereof for the purpose of streamlining the disclosure and aiding in the understanding of one or more of the various inventive aspects. This method of disclosure, however, is not to be interpreted as reflecting an intention that any claim require more features than are expressly recited in that claim. Moreover, any components, features, or steps illustrated and/or described in a particular embodiment herein can be applied to or used with any other embodiment(s). Further, no component, feature, step, or group of components, features, or steps are necessary or indispensable for each embodiment. Thus, it is intended that the scope of the inventions herein disclosed and claimed below should not be limited by the particular embodiments described above, but should be determined only by a fair reading of the claims that follow.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited.

Unless otherwise defined, all terms (including technical and scientific terms) used herein have the same meaning as commonly understood by one of ordinary skill in the art to which example embodiments belong. It be further understood that terms, such as those defined in commonly used dictionaries, should be interpreted as having a meaning that is consistent with their meaning in the context of the relevant art and not be interpreted in an idealized or overly formal sense unless expressly so defined herein.

The spatially relative terms "outer," "inner," "upper," "lower," "below," "above," "vertical," "horizontal," and similar terms, may be used herein for ease of description to describe the relations between one element or component and another element or component as illustrated in the drawings. It be understood that the spatially relative terms are intended to encompass different orientations of the device in use or operation, in addition to the orientation depicted in the drawings. For example, in the case where a device shown in the drawing is turned over, the device positioned "below" or "beneath" another device may be placed "above" another device. Accordingly, the illustrative term "below" may include both the lower and upper positions. The device may also be oriented in the other direction, and thus the spatially relative terms may be interpreted differently depending on the orientations.

Unless otherwise expressly stated, comparative and/or quantitative terms, such as "less," "more," "greater," and the like, are intended to encompass the concepts of equality. For example, "less" can mean not only "less" in the strictest mathematical sense, but also, "less than or equal to."

## Claims

1. An alignment device comprising:
an elongate rod (94);
a sealing hub (95) coupled to a proximal end of the elongate rod and configured to form a fluid-tight seal within a portion of a percutaneous-access device (200); and
an alignment structure (92) coupled to the sealing hub and having a form configured to engage with a component of an instrument manipulator assembly of a robotic system, wherein the alignment structure has a concave proximal surface conforming to an outer surface of the instrument manipulator assembly.

2. The alignment device of claim 1, wherein the elongate rod has a blunt distal end.

3. The alignment device of claim 1, wherein a distal end of the elongate rod includes a flat axial face portion and a rounded circumferential edge portion.

4. The alignment device of claim 1, wherein the alignment structure has a vertical-alignment lip (96) that is inwardly-projecting and circumferential with respect to a curvature of the concave proximal surface, and optionally wherein the vertical-alignment lip is configured to be placed against at least one of an upward-facing surface or a downward-facing surface of the component of the instrument manipulator assembly when the concave proximal surface is placed against the outer surface of the instrument manipulator assembly.

5. The alignment device of claim 1, wherein the alignment structure includes one or more alignment markings (97) indicating an alignment position of the alignment structure relative to at least one component of the instrument manipulator assembly.

6. The alignment device of claim 1, wherein the sealing hub has external threading (89) configured to engage with internal threading of the percutaneous-access device.

7. The alignment device of claim 1, wherein the alignment structure is configured to be attached to the sealing hub from a state separate from the sealing hub, and optionally wherein the alignment structure comprises a distally-projecting male connector configured to be coupled to the sealing hub.

8. The alignment device of claim 1, wherein the alignment structure is axially rotatable relative to the sealing hub.

9. The alignment device of claim 1, wherein the sealing hub comprises a tapered surface (93) configured to be placed against a corresponding interior surface of the percutaneous-access device to form the fluid-tight seal.

10. A robotic system comprising:
a robotic arm (12);
an instrument manipulator assembly associated with a distal portion of the robotic arm;
a percutaneous-access device (200) comprising an elongate shaft forming an access lumen; and
the alignment device of claim 1, wherein the elongate rod is configured to be disposed at least partially within the access lumen.

11. The robotic system of claim 10, wherein the instrument manipulator assembly includes an adapter component (8), the outer surface being associated with a rotating plate of the adapter component, and optionally wherein:
the adapter component is coupled to a drape that is configured to provide a sterile barrier between the robotic arm and an instrument of the instrument manipulator assembly that is coupled to the adapter component, or
the robotic system further comprises a catheter assembly including a catheter coupled to a catheter handle configured to be attached to the adapter component.

12. The robotic system of claim 10, wherein the alignment structure is secured to the alignment device on a proximal side of the sealing hub.

13. The robotic system of claim 10, wherein the robotic arm is configured to move along an alignment path that is based on kinematic data relating to a position of the robotic arm when the instrument manipulator assembly is engaged with the alignment structure of the alignment device.

14. A percutaneous-access kit (150) comprising:
a catheter-alignment device (90) for use in an alignment process in which an alignment path and/or position is determined for a catheter relative to a percutaneous-access sheath (200), the catheter-alignment device including an elongate rod (94), a proximal hub (95) and a proximal alignment structure (92);
wherein the proximal hub is configured to form a proximal fluid seal between the proximal hub and a hub of the percutaneous-access sheath,
wherein the alignment structure has a concave proximal surface conforming to an outer surface of an instrument manipulator assembly of a robotic system, and
wherein the elongate rod and the proximal hub are assembled together in the percutaneous-access kit.

15. The percutaneous-access kit of claim 14, wherein the percutaneous-access kit further comprises:
a catheter (48) coupled to a catheter handle; and
the percutaneous-access sheath,
wherein the percutaneous-access sheath has an access lumen, and optionally wherein:
the alignment structure is separate from the elongate rod and the proximal hub in the percutaneous-access kit.

## Patentansprüche

1. Ausrichtungsvorrichtung, umfassend:
eine längliche Stange (94);
eine Dichtungsnabe (95), die mit einem proximalen Ende der länglichen Stange gekoppelt und konfiguriert ist, um eine fluiddichte Abdichtung innerhalb eines Abschnitts einer perkutanen Zugangsvorrichtung (200) auszubilden; und
eine Ausrichtungsstruktur (92), die mit der Dichtungsnabe gekoppelt ist und eine Form aufweist, die konfiguriert ist, um mit einer Komponente einer Instrumentenmanipulatoranordnung eines Robotersystems in Eingriff zu stehen, wobei die Ausrichtungsstruktur eine konkave proximale Oberfläche aufweist, die mit einer Außenoberfläche der Instrumentenmanipulatoranordnung übereinstimmt.

2. Ausrichtungsvorrichtung nach Anspruch 1, wobei die längliche Stange ein stumpfes distales Ende aufweist.

3. Ausrichtungsvorrichtung nach Anspruch 1, wobei ein distales Ende der länglichen Stange einen flachen axialen Flächenabschnitt und einen abgerundeten umlaufenden Kantenabschnitt einschließt.

4. Ausrichtungsvorrichtung nach Anspruch 1, wobei die Ausrichtungsstruktur eine vertikale Ausrichtungslippe (96) aufweist, die nach innen vorsteht und hinsichtlich einer Krümmung der konkaven proximalen Oberfläche umlaufend ist, und gegebenenfalls wobei die vertikale Ausrichtungslippe konfiguriert ist, um gegen mindestens eine von einer nach oben weisenden Oberfläche oder einer nach unten weisenden Oberfläche der Komponente der Instrumentenmanipulatoranordnung platziert zu werden, wenn die konkave proximale Oberfläche gegen die Außenoberfläche der Instrumentenmanipulatoranordnung platziert wird.

5. Ausrichtungsvorrichtung nach Anspruch 1, wobei die Ausrichtungsstruktur eine oder mehrere Ausrichtungsmarkierungen (97) einschließt, die eine Ausrichtungsposition der Ausrichtungsstruktur relativ zu mindestens einer Komponente der Instrumentenmanipulatoranordnung angeben.

6. Ausrichtungsvorrichtung nach Anspruch 1, wobei die Dichtungsnabe ein Außengewinde (89) aufweist, das konfiguriert ist, um mit eine, Innengewinde der perkutanen Zugangsvorrichtung in Eingriff zu stehen.

7. Ausrichtungsvorrichtung nach Anspruch 1, wobei die Ausrichtungsstruktur konfiguriert ist, um aus einem Zustand, der von der Dichtungsnabe getrennt ist, an der Dichtungsnabe befestigt zu werden, und gegebenenfalls wobei die Ausrichtungsstruktur einen distal vorstehenden männlichen Verbinder umfasst, der konfiguriert ist, um mit der Dichtungsnabe gekoppelt zu werden.

8. Ausrichtungsvorrichtung nach Anspruch 1, wobei die Ausrichtungsstruktur relativ zu der Dichtungsnabe axial drehbar ist.

9. Ausrichtungsvorrichtung nach Anspruch 1, wobei die Dichtungsnabe eine verjüngte Oberfläche (93) umfasst, die konfiguriert ist, um an eine entsprechende innere Oberfläche der perkutanen Zugangsvorrichtung angelegt zu werden, um die fluiddichte Abdichtung auszubilden.

10. Robotersystem, umfassend:
einen Roboterarm (12);
eine Instrumentenmanipulatoranordnung, die einem distalen Abschnitt des Roboterarms zugeordnet ist;
eine perkutane Zugangsvorrichtung (200), umfassend einen länglichen Schaft, der ein Zugangslumen ausbildet; und
die Ausrichtungsvorrichtung nach Anspruch 1, wobei die längliche Stange konfiguriert ist, um mindestens teilweise innerhalb des Zugangslumens eingerichtet zu sein.

11. Robotersystem nach Anspruch 10, wobei die Instrumentenmanipulatoranordnung eine Adapterkomponente (8) einschließt, wobei die Außenoberfläche einer sich drehenden Platte der Adapterkomponente zugeordnet ist, und gegebenenfalls wobei:
die Adapterkomponente mit einem Abdecktuch gekoppelt ist, das konfiguriert ist, um eine sterile Barriere zwischen dem Roboterarm und einem Instrument der Instrumentenmanipulatoranordnung bereitzustellen, das mit der Adapterkomponente gekoppelt ist, oder
das Robotersystem ferner eine Katheteranordnung umfasst, die einen Katheter einschließt, der mit einem Kathetergriff gekoppelt ist, der konfiguriert ist, um an der Adapterkomponente befestigt zu werden.

12. Robotersystem nach Anspruch 10, wobei die Ausrichtungsstruktur an der Ausrichtungsvorrichtung auf einer proximalen Seite der Dichtungsnabe gesichert ist.

13. Robotersystem nach Anspruch 10, wobei der Roboterarm konfiguriert ist, um sich entlang eines Ausrichtungspfads zu bewegen, der auf kinematischen Daten basiert, die sich auf eine Position des Roboterarms beziehen, wenn die Instrumentenmanipulatoranordnung mit der Ausrichtungsstruktur der Ausrichtungsvorrichtung in Eingriff steht.

14. Perkutanes Zugangskit (150), umfassend:
eine Katheter-Ausrichtungsvorrichtung (90) zur Verwendung in einem Ausrichtungsprozess, in dem ein Ausrichtungspfad und/oder eine Position für einen Katheter relativ zu einer perkutanen Zugangshülle (200) bestimmt wird, wobei die Katheter-Ausrichtungsvorrichtung eine längliche Stange (94), eine proximale Nabe (95) und eine proximale Ausrichtungsstruktur (92) einschließt;
wobei die proximale Nabe konfiguriert ist, um eine proximale Fluiddichtung zwischen der proximalen Nabe und einer Nabe der perkutanen Zugangshülle auszubilden,
wobei die Ausrichtungsstruktur eine konkave proximale Oberfläche aufweist, die einer Außenoberfläche mit einer Instrumentenmanipulatoranordnung eines Robotersystems übereinstimmt, und
wobei die längliche Stange und die proximale Nabe in dem perkutanen Zugangskit zusammengebaut sind.

15. Perkutanes Zugangskit nach Anspruch 14, wobei das perkutane Zugangskit ferner umfasst:
einen Katheter (48), der mit einem Kathetergriff gekoppelt ist; und
die perkutane Zugangshülle,
wobei die perkutane Zugangshülle ein Zugangslumen aufweist, und gegebenenfalls wobei:
die Ausrichtungsstruktur von der länglichen Stange und der proximalen Nabe in dem perkutanen Zugangskit getrennt ist.

## Revendications

1. Dispositif d'alignement comprenant :
une tige allongée (94) ;
un moyeu d'étanchéité (95) accouplé à une extrémité proximale de la tige allongée et conçu pour former un joint étanche aux fluides au sein d'une partie d'un dispositif d'accès percutané (200) ; et
une structure d'alignement (92) accouplée au moyeu d'étanchéité et ayant une forme conçue pour venir en prise avec un composant d'un ensemble manipulateur d'instrument d'un système robotique, dans lequel la structure d'alignement a une surface proximale concave conforme à une surface externe de l'ensemble manipulateur d'instrument.

2. Dispositif d'alignement selon la revendication 1, dans lequel la tige allongée a une extrémité distale émoussée.

3. Dispositif d'alignement selon la revendication 1, dans lequel une extrémité distale de la tige allongée comporte une partie face axiale plate et une partie bord circonférentiel arrondi.

4. Dispositif d'alignement selon la revendication 1, dans lequel la structure d'alignement a une lèvre d'alignement vertical (96) qui fait saillie vers l'intérieur et est circonférentielle par rapport à une courbure de la surface proximale concave, et éventuellement dans lequel la lèvre d'alignement vertical est conçue pour être placée contre au moins l'une parmi une surface orientée vers le haut ou une surface orientée vers le bas du composant de l'ensemble manipulateur d'instrument lorsque la surface proximale concave est placée contre la surface externe de l'ensemble manipulateur d'instrument.

5. Dispositif d'alignement selon la revendication 1, dans lequel la structure d'alignement comporte un ou plusieurs repères d'alignement (97) indiquant une position d'alignement de la structure d'alignement par rapport à au moins un composant de l'ensemble manipulateur d'instrument.

6. Dispositif d'alignement selon la revendication 1, dans lequel le moyeu d'étanchéité a un filetage externe (89) conçu pour venir en prise avec un filetage interne du dispositif d'accès percutané.

7. Dispositif d'alignement selon la revendication 1, dans lequel la structure d'alignement est conçue pour être fixée au moyeu d'étanchéité à partir d'un état séparé du moyeu d'étanchéité, et éventuellement dans lequel la structure d'alignement comprend un connecteur mâle à projection distale conçu pour être accouplé au moyeu d'étanchéité.

8. Dispositif d'alignement selon la revendication 1, dans lequel la structure d'alignement est axialement rotative par rapport au moyeu d'étanchéité.

9. Dispositif d'alignement selon la revendication 1, dans lequel le moyeu d'étanchéité comprend une surface effilée (93) conçue pour être placée contre une surface intérieure correspondante du dispositif d'accès percutané pour former le joint étanche aux fluides.

10. Système robotique, comprenant :
un bras robotique (12) ;
un ensemble manipulateur d'instrument associé à une partie distale du bras robotique ;
un dispositif d'accès percutané (200) comprenant une tige allongée formant une lumière d'accès ; et
le dispositif d'alignement selon la revendication 1, dans lequel la tige allongée est conçue pour être disposée au moins partiellement à l'intérieur de la lumière d'accès.

11. Système robotique selon la revendication 10, dans lequel l'ensemble manipulateur d'instrument comporte un composant adaptateur (8), la surface externe étant associée à une plaque rotative du composant adaptateur, et éventuellement dans lequel :
le composant adaptateur est accouplé à un champ qui est conçu pour fournir une barrière stérile entre le bras robotique et un instrument de l'ensemble manipulateur d'instrument qui est accouplé au composant adaptateur, ou
le système robotique comprend en outre un ensemble cathéter comportant un cathéter accouplé à une poignée de cathéter conçue pour être attachée au composant adaptateur.

12. Système robotique selon la revendication 10, dans lequel la structure d'alignement est fixée au dispositif d'alignement sur un côté proximal du moyeu d'étanchéité.

13. Système robotique selon la revendication 10, dans lequel le bras robotique est conçu pour se déplacer le long d'une trajectoire d'alignement qui est basée sur des données cinématiques relatives à une position du bras robotique lorsque l'ensemble manipulateur d'instrument est en prise avec la structure d'alignement du dispositif d'alignement.

14. Trousse d'accès percutané (150) comprenant :
un dispositif d'alignement de cathéter (90) destiné à être utilisé dans un processus d'alignement dans lequel une trajectoire d'alignement et/ou une position sont déterminés pour un cathéter par rapport à une gaine d'accès percutané (200), le dispositif d'alignement de cathéter comportant une tige allongée (94), un moyeu proximal (95) et une structure d'alignement proximale (92) ;
dans laquelle le moyeu proximal est conçu pour former un joint fluidique proximal entre le moyeu proximal et un moyeu de la gaine d'accès percutané,
dans laquelle la structure d'alignement a une surface proximale concave conforme à une surface extérieure d'un ensemble manipulateur d'instrument d'un système robotique, et
dans laquelle la tige allongée et le moyeu proximal sont assemblés ensemble dans la trousse d'accès percutané.

15. Trousse d'accès percutané selon la revendication 14, dans laquelle la trousse d'accès percutané comprend en outre :
un cathéter (48) accouplé à une poignée de cathéter ; et
la gaine d'accès percutané,
dans laquelle la gaine d'accès percutané a une lumière d'accès, et éventuellement dans laquelle :
la structure d'alignement est séparée de la tige allongée et du moyeu proximal dans la trousse d'accès percutané.
